# EUROPEAN PATENT SPECIFICATION

(11) **EP 1 024 806 B1**
(45) Date of publication and mention of the grant of the patent: **08.06.2005**
(21) Application number: 98950843.7
(22) Date of filing: 02.10.1998
(51) Int. Cl.: A61K 31/395, A61K 38/02, A61K 39/395, G01N 33/74

(54) **USE OF NON-IMMUNOSUPPRESSIVE COMPOUNDS FOR PROMOTING NERVE REGENERATION**
VERWENDUNG VON NICHTIMMUNUNTERDRUCKENDE VERBINDUNGEN ZUR FÖRDERUNG VON NERVENREGENERATION
UTILISATION DE COMPOSES NON-IMMUNOSUPPRESSIFS FAVORISANT LA REGENERATION DES TISSUS NERVEUX

(30) Priority: 24.10.1997 US 956691
(43) Date of publication of application: 09.08.2000
(73) Proprietor: OREGON HEALTH SCIENCES UNIVERSITY, Portland, OR 97201-3098 (US)
(72) Inventor: GOLD, Bruce, Gordon, West Linn, OR 97068 (US)
(74) Representative: Gowshall, Jonathan Vallance
(86) International application number: PCT/US1998/020658
(87) International publication number: WO 1999/021552

(56) References cited:
- WO-A-96/40140
- WO-A-97/18828
- D.F. SMITH ET AL.: "Progesterone receptor structure and function altered by geldanamycin, an hsp90-binding agent." MOL. CELL. BIOL., vol. 15, no. 12, 1995, pages 6804-6812, XP002097518
- B. SEGNITZ ET AL.: "The function of steroid hormone receptor is inhibited by the hsp90-specific compound." J. BIOL. CHEM., vol. 272, no. 30, 1997, pages 18694-18701, XP002097519

## Description

### FIELD OF THE INVENTION

This invention concerns neurotrophic compounds useful in the treatment of neurological injury and disease.

### BACKGROUND OF THE INVENTION

Following traumatic or mechanically induced axonal degeneration in the peripheral nervous system, axonal regeneration often ensues, resulting in functional recovery. However, the rate of axonal elongation (3-4 mm/day) is slow, and sometimes does not result in recovery of full neurological function. If neurological function is restored, recovery usually occurs in weeks or months, depending upon the distance between the site of injury and the target tissue. Therapies that speed regeneration over long distances would be highly beneficial to patients and would significantly reduce health care costs.

Other neurological conditions result from dysfunction of neurons in the peripheral or central nervous systems that is caused by chronic disease or injury. Chronic disease processes can permanently and progressively damage the nervous system, and (particularly in the central nervous system) usually results in permanent loss of function. Such loss of neurological function is a major cause of physical incapacitation and death throughout the world.

The immunosuppressant drug FK506 (USAN tacrolimus; Prograf®) induces immunosuppression by binding the immunophilin FKBP-12. This binding prevents calcineurin from dephosphorylating the transcription factor NF/AT (nuclear factor of activated T-cells), which blocks translocation of calcineurin into the nucleus, and prevents a receptor-mediated increase in the synthesis and secretion of cytokines, such as interleukin-2 (IL-2), which are required for T-cell proliferation. FK506 has also been found to speed functional recovery and axonal regeneration in the rat in a dose-dependent manner following a sciatic nerve crush lesion.

U.S. Patent No. 5,654,332 (Armistead et al.) discusses immunosuppressive FK506 analogs that bind FKBP-12, and are said to stimulate neurite outgrowth in the presence of NGF. The neurotrophic activity of these FKBP-12 binding compounds was said to be "directly related to their affinity for FKBP-12 and their ability to inhibit FKBP-12 rotamase activity" (id. at col. 7, lines 47-50). Rotamase activity measures peptidylisomerase cis-trans isomerization, and inhibition of this activity has been accepted as an indication of the immunosuppresant and neurotrophic activity of therapeutic agents. See U.S. Patent No. 5,614,547 (Hamilton et al.).

Systemic administration of two synthetic FK506 analogs that bind FKBP-12 but that do not inhibit calcineurin activity (and which are not immunosuppressants) have been reported to increase the size of myelinated fibers (Gold et al., *Exp. Neurol.* 147:269-278, 1997; Steiner et al., *Nature Medicine* 3:1-8, 1997; Steiner et al., *Proc. Natl. Acad. Sci. USA* 94:2019-2024, 1997). It has also been reported that androgens and estrogens stimulate facial nerve regeneration in hamsters (e.g. Tanzer and Jones, *Exp. Neurol.* 146:258-264, 1997).

Many of the compounds previously shown to stimulate nerve regeneration have undesired side-effects, such as immunosuppression (FK506 and analogs that retain immunosuppressant activity) or androgenic or estrogenic stimulation. There is therefore a need to provide a class of nerve growth stimulating compounds that are well tolerated by subjects who take them.

### SUMMARY OF THE INVENTION

The mechanism by which FK506 and other analogs induces nerve growth stimulation has previously been misunderstood, which has been an obstacle to the development of new drugs for this purpose.

The present invention takes advantage of the surprising discovery that nerve growth stimulation is promoted by disruption of the mature steroid receptor complex, and not by interaction with FKBP-12, as was previously thought. Disruption of the complex can include inhibition of physical assembly, promotion of disassembly, or functional interference with the steroid receptor complex, for example the mature steroid receptor complex, or a less mature form of the complex that is a predecessor to the mature complex. In view of the discovery of the biochemical mechanism by which neurite outgrowth is promoted, the present invention includes assays according to the claims for selecting new compounds that may have activity in promoting nerve growth. Such assays may include determining if a test compound, other than a steroid ligand such as an androgen or an estrogen, disrupts assembly of the steroid receptor complex, and selecting a compound that disrupts assembly of the steroid receptor complex. Examples of specific classes of compounds that can be screened include geldanamycin and its structural analogs, rapamycin and its structural analogs, and FK506 and its structural analogs. Compounds selected by this assay for further investigation may be tested in additional assays to measure actual neurite outgrowth induced by the compound. The invention also includes neurotrophic compounds identified by the assay for disruption of the steroid receptor complex.

The disclosure also includes methods of stimulating nerve cell growth in a subject by administering to the subject a compound (including a compound discovered by the assay) that disrupts assembly or function of the steroid receptor complex, for example of the mature steroid receptor complex, (for example by inhibiting association or promoting dissociation), wherein the compound is other than a ligand for the steroid hormone binding portion of the steroid receptor complex (such as an androgen or an estrogen), and in some specific embodiments does not bind with high affinity to FKBP-12. In particular embodiments, the compound is administered to disrupt association of a p23 component of the steroid receptor complex with an hsp-90 component or disrupt association of FKBP-52 with hsp-90. In other embodiments, the compound is administered to competitively bind with ATP at an amino terminal ATP binding site of hsp-90, for example at a geldanamycin binding site of the steroid receptor complex. These methods include administration of a benzoquinone ansamycin, such as geldanamycin or a structural analog or mimetic thereof, or an anti-FKBP-52 antibody. The method can also include administering a second neurotrophic factor, other than the compound that disrupts association of the steroid receptor complex.

The method is useful in the treatment of animals (including mammals such as humans) having a neurological condition associated with neuronal dysfunction caused by disease or injury to neurons in either the central or peripheral nervous systems. The use of the compounds or compositions, according to the present invention as defined in the claims, is by administering to the animal in a therapeutically effective neurotrophic amount to bind to the mature steroid receptor complex (for example at a geldanamycin binding site of hsp-90) to disrupt association of the mature steroid receptor complex, and promote neurite outgrowth from neurons. The method can also be used in association with procedures such as a surgical nerve graft, or other implantation of neurological tissue, to promote healing of the graft or implant, and promote incorporation of the graft or implant into adjacent tissue.

This invention also includes use according to the claims of pharmaceutical compounds that disrupt assembly of a steroid receptor complex, such as compounds that are not a ligand for the steroid hormone binding portion of the steroid receptor complex. These compounds can be rapamycin or FK506 analogs, but more particular embodiments of the compound may have low rotamase inhibition activity, may be other than an FK506 or rapamycin analog, may not bind with high affinity to FKBP-12, or are not immunosuppressive. In particular embodiments, the compound specifically disrupts formation of the steroid receptor complex (for example the mature steroid receptor complex) either by inhibiting association or promoting dissociation of the steroid receptor complex, for example by disrupting association of a p23 component of the steroid receptor complex with an hsp-90 component, or disrupting association of FKBP-52 with hsp-90, or inhibiting interaction of p23, FKBP-52 or hsp-90 with the complex.

Certain embodiments of the compound competitively bind with ATP at an amino terminal ATP binding site of hsp-90, which is also the binding site for geldanamycin binding to the steroid receptor complex. In particular embodiments the compound is a benzoquinone ansamycin that binds to a geldanamycin binding site of hsp-90, such as geldanamycin or a structural analog or mimetic thereof. In other embodiments, the compound is an anti-FKBP-52 antibody, or another agent that specifically causes FKBP-52 to dissociate from hsp-90 of the steroid receptor complex.

The compound can be incorporated into a pharmaceutical composition, which can also include another neurotrophic factor, such as NGF, IGF-1, αFGF, βFGF, PDGF, BDNF, CNTF, GDNF, NT-3, NT 4/5, and mixtures thereof, or a steroid hormone that is a ligand of the steroid receptor complex (such as an estrogen, an androgen or a corticosteroid such as dexamethasone).

In a more specific aspect of the use of the invention as defined in the claims, the compound is a nerve growth stimulating amount of an agent that binds to a polypeptide of a steroid receptor complex other than a steroid hormone binding portion of the complex, the agent being selected from the group consisting of an FK506 analog having low binding affinity for FKBP-12 and low rotamase activity, for example a benzoquinone ansamycin and structural analogs thereof, a peptide comprising a sequence of a selected polypeptide component of the complex at a site of interaction between the selected component and another polypeptide component of the complex, an antibody, and combinations thereof, wherein the agent disrupts assembly or interferes with function of the steroid receptor complex by causing p23 or FKBP-52 dissociation from the complex, or inhibiting p23 or FKBP-52 association with the complex, or inhibiting interaction of p23, FKBP-52 or hsp-90 with the complex.

Compounds of the use of the present invention need not have singificant calcineurin inhibition or rotamase inhibition. The compounds may have an IC₅₀ for rotamase inhibtion of greater than I nM, for example greater than 10 nM, 25 nM, or even 50-100 nM.

The foregoing and various features and advantages of the invention will become more apparent from the following detailed description and accompanying drawings.

### BRIEF DESCRIPTION OF THE FIGURES

FIG. 1 is a schematic diagram illustrating receptor-hsp-90 heterocomplex assembly of the steroid receptor complex.
FIG. 2 is a schematic diagram illustrating the mature steroid receptor complex, and the binding sites of some of the agents of the present invention that promote nerve growth.
FIGS. 3-17 are cumulative histograms showing neurite outgrowth length, in SH-SY5Y cells in the presence of nerve growth factor NGF alone (10 ng/ml) and in the presence of NGF (10 ng/ml) and the concentrations of other agents listed on each histogram. Curves shifted to the right indicate longer processes, and greater neurite length outgrowth.

### DETAILED DESCRIPTION OF A PREFERRED EMBODIMENT

Steroid receptors are part of a superfamily of molecules that regulate gene expression by direct interaction with the upstream region of specific structural genes. It is essential to hormone action that a receptor must be able to assume both an active and an inactive state. This regulation is accomplished by association of the receptor (the steroid ligand binding component) with a multimeric complex of chaperone proteins, such as heat shock proteins (hsp-90), p23 and FKBP-52, which form the steroid receptor complex (SRC). When the steroid receptor binds its ligand, the receptor is activated, the chaperone proteins of the SRC are dissociated, and a DNA binding domain of the receptor is exposed for interaction with gene regulatory sequences. Members of the steroid receptor family that are regulated in this fashion include mineralocorticoids (such as aldosterone), glucocorticoids (such as dexamethasone), progestins (such as progesterone), androgens (such as testosterone), and estrogens (including estrogen, -estriol and -estradiol).

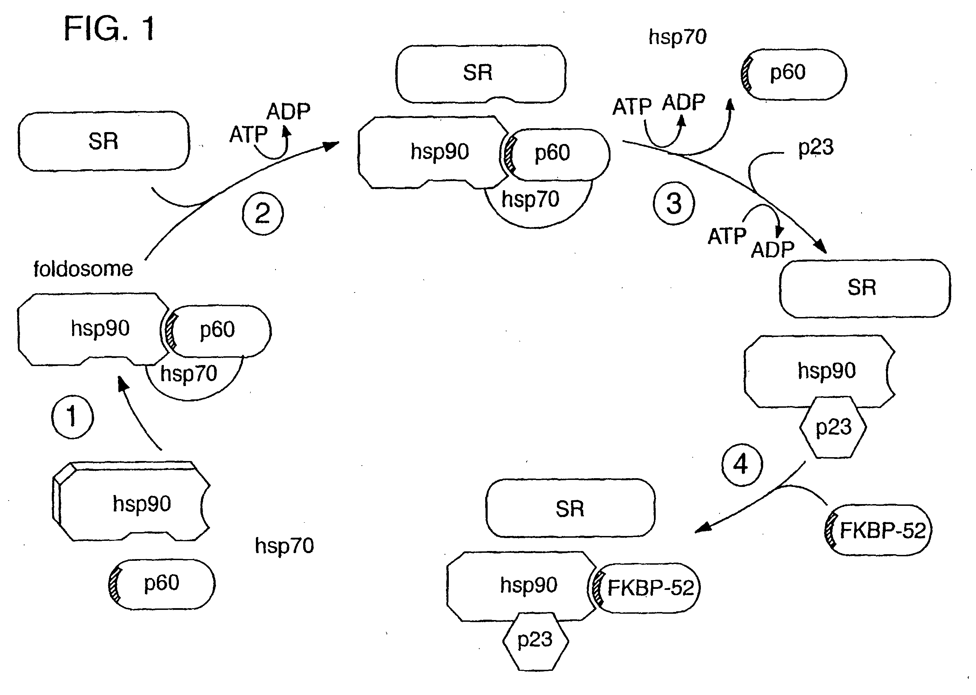

A model of steroid receptor complex assembly is shown in FIG. 1. ATP-dependent association of the steroid receptor SR with the hsp-90/p-60/hsp-70 folding complex (foldosome) yields an intermediate (SR/hsp-90/p-60/hsp-70 ) complex that is unstable in the absence of p23 or molybdate. Once p23 has associated with the complex, and hsp-90 binds tetratricopeptide repeat (TPR) domain proteins, such as the immunophilins (e.g. FKBP-52 and CyP40), the nearly mature, metastable SRC is formed (SR/hsp-90/p23/FKBP-52). In FIG. 1, the TPR domain is indicated by the solid black crescent to which FKBP-52 is bound. Assembly of the mature steroid receptor complex is the last step shown in FIG. 1, in which the complex of chaperone proteins hsp-90/p23/FKBP-52 is assembled with the steroid receptor SR.

The immunophilins are a highly conserved family of chaperone proteins that are known to be mediators of immunosuppressant drug activity. The best characterized immunophilin is FKBP-12, which interacts with the immunosuppressant drug FK-506 in T lymphocytes, to prevent calcineurin from dephosphorylating the nuclear factor of activated T-cells (NF/AT), thereby blocking synthesis and secretion of cytokines required for immune function. Immunophilins have peptidylisomerase (PPIase) activity, and inhibitors of this activity can be detected with a rotamase assay which measures inhibition of *cis-trans* isomerization of the peptidylprolyl. However, FKBP-12 immunosupression is not mediated by an ability to inhibit rotamase activity. Rotamase activity has nonetheless been accepted as an indication of immunosuppressant activity of immunophilins, even though it does not measure the dephosphorylation of calcineurin activity by which immunosuppression is actually mediated. Previous researchers had taken advantage of the rotamase assay to look for FKBP-12 binding drugs that would promote nerve regeneration (as FK506 had been found to do).

The present invention takes advantage of the surprising finding that disrupting assembly of the SRC, and not binding to FKBP-12, is what promotes nerve regeneration. Hence previous efforts to find FKBP-12 analogs that promote nerve regeneration by measuring rotamase or immunosuppressive activity was misdirected, and was likely to find drugs that had unwanted side effects (such as immunosuppression and cardiomyopathy). The present invention has taken advantage of the discovery of the actual biological mechanism by which nerve regeneration is promoted to provide a superior assay for finding new neurotrophic drugs that are superior to those in the prior art.

One such new compound is geldanamycin, a benzoquinone ansamycin antibiotic, which binds in a pharmacologically specific manner to hsp-90 (Whitesell et al., *Proc. Natl. Acad. Sci. USA* 91:8324-8328, 1994) and inhibits association of the p23 component of the heterocomplex assembly system with hsp-90 (Johnson and Toft, *Mol. Endocrinol.* 9:670-678, 1995). Geldanamycin thereby promotes dissociation of a steroid receptor complex. and blocks reassembly of the hormone-responsive form of the complex, preventing hormone activation and ultimately resulting in the degradation of the hormone receptor. Geldanamycin blocks assembly of the progesterone receptor (PR) complex (Smith et al., *Mol. Cell. Biol*. 15:6804-6812, 1995) and of the glucocorticoid receptor (GR) complex (Czar et al., *Biochem.* 36:7776-7785, 1997) at an intermediate stage of assembly where the hormone binding domain is not properly folded and therefore cannot bind steroid with high affinity (for example, does not bind steroid ligand that is present in concentrations of less than about 10 nM). Geldanamycin also is known to act on estrogen and androgen hormone receptors (Smith et al., *Mol. Cell. Biol.* 15:6804-6812, 1995; Nair et al., *Cell Stress and Chaperones* 1:337-250, 1996). Transformation of GR and PR as measured either by 9S to 4S conversion, or by acquisition of DNA-binding activity, is correlated with dissociation of steroid receptors from hsp-90 (see, e.g., Meshinchi et al., *J. Biol. Chem*. 265:4863-4870, 1990; Kost et al., *Mol. Cell. Biol*. 9:3829-3838, 1989).

Another class of neurotrophic agents that have been found in accordance with the invention are those that act at the FKBP-52 component of the SRC. It has surprisingly been found that the action of the neurotrophic immunosuppressant FK506 is via an interaction with FKBP-52, which induces a conformational change in hsp-90, enabling dissociation of p23 from hsp-90, thereby interfering with assembly of the mature SRC. The present invention also includes an anti-FKBP-52 antibody which also inhibits assembly of the mature SRC. Binding of FK506 to GR-associated FKBP-52 causes increased nuclear translocation of GR in response to dexamethasone and potentiation of GR-mediated gene expression (Sanchez and Ning, *METHODS: A Companion to Meth. Enzymol*. 9:188-200, 1996).

FKBP-52 and CyP40 bind directly to hsp-90, and CyP40 competes for FKBP-52 binding to hsp-90 and vice versa. CyP40 is an example of a protein targeted by cyclosporin A (CsA) and its analogs. These immunophilins bind hsp-90 in a mutually exclusive fashion, leading to the formation of separate CyP40-hsp-90 and FKBP-52-hsp-90 complexes (Ratajczak and Carrello, *J. Biol. Chem*. 271:2961-2965, 1996). Immunophilins such as FKBP-52, CyP40 and PP5 and non-immunophilin proteins such as p60 and Mas70p, have one or more tetratricopeptide repeat (TPR) domains (Ratajczak et al., *J. Biol. Chem*. 268:13187-13192, 1993) that bind to the TPR-binding domain of hsp-90. An increased number of TPR domains in a protein appears to correlate with increased hsp-90-binding affinity. Hence peptides having one or more TPR domains would be expected to have increased hsp-90 binding affinity, and would interfere with FKBP-52 association with hsp-90, which is required for assembly of the mature steroid receptor complex.

For example, binding of both FKBP-52 and CyP40 to hsp-90 is competively inhibited by a purified fragment of human CyP40 comprising its three TPR domains, and by a fragment of rat PP5 comprising its four TPR domains (reviewed in Pratt and Toft, *Endocrine Rev*, 18:306-360, 1997). Such purified fragments, or other peptides such as PP5, p60 and Mas70, containing one or more TPR domains (particularly two, three or more TPR domains) are therefore suitable for interfering with assembly or function of the steroid receptor complex, and are included within the scope of this invention.

The effects of geldanamycin and the other neurotrophic agents are believed to result from binding of these compounds to components of steroid receptor complexes, causing the dissociation of hsp-90 from the steroid receptor complex either directly (by binding to hsp-90 or interfering with the binding of hsp-90 to the steroid receptor) or indirectly (by binding to a polypeptide such as FKBP-52 that itself binds to hsp-90, or a polypeptide that binds to p23), or alternatively by preventing association of hsp-90 or p23 with the steroid receptor complex. Interference with the ability of hsp-90 to complex with and perform its chaperone function for other hsp-90 substrate proteins is believed to also be responsible for or contribute to the observed stimulation of nerve regeneration by FK506 and/or geldanamycin.

Specific embodiments of the neurotrophic compounds of this disclosure can be substantially free of calcineurin inhibition, and can have low rotamase inhibition, for example an IC₅₀ of greater than about 1 nM, or even 5 or 10 nM.

### Abbreviations and Definitions

**AR:** Androgen Receptor
**ER:** Estrogen receptor
**GR:** Glucocorticoid receptor
**PR:** Progesterone receptor
**SRC:** Steroid receptor complex. A multiprotein complex associated with any steroid receptor, including, but not limited to, the progesterone receptor, glucocorticoid receptor, estrogen receptor, androgen receptor, and mineralocorticoid receptor.
**TPR:** Tetratricopeptide repeats are Domain III of FKBP-52; TPRs were first identified by Sikorski et al., Cell 60:307-317, 1990, as degenerate consensus sequences of 34 amino acids.
**Mimetic:** A biological compound (such as a peptide) that mimics the effect of a pharmaceutical, for example a peptide that mimics the effect of a benzoquinone ansamycin by binding to a geldanamycin binding site on hsp-90.
**Ligand for the steroid hormone binding portion of the steroid receptor complex:** An already recognized ligand for the receptor subtype. For example, dexamethasone for the GR, estrogen for the ER, testosterone for the AR, progesterone for the PR.
**Immunophilins:** A highly conserved family of chaperone proteins that have PPlase activity, producing cis-trans isomerization. The immunophilins are divided into low molecular weight (less than 40kD) and high molecular weight (40-65 kD) immunophilins. The high molecular weight immunophilins (e.g., FKBP-52), in contrast to FKBP-12. contain three or more tetratricopeptide repeats (TPRs) which mediate binding to hsp-90. The immunophilins may be subdivided into two classes on the basis of their abilty to bind either cyclosporin A (cyclophilins) or FK506 and rapamycin (the FK binding proteins, FKBPs). Members of the FKBP family of immunophilins include FKBP-12, FKBP-13, FKBP-25, FKBP-52 (also referred to as FKBP-59), and FKBP-65. PP5 is also considered a member of this family, because it binds FK506 weakly, as reported by Silverstein et al., *J. Biol. Chem*. 272:16224-16230, 1997.
**NGPA:** Nerve growth promoting agent. A "nerve growth promoting agent" or NGPA is defined as a substance that binds to a polypeptide component of a steroid receptor complex, such components including but not limited to hsp-90 and FKBP-52, and promotes nerve regeneration, without limitation to a particular mechanism of action. In particular embodiments, the NGPA does not bind FKBP-12 (or binds it with low affinity, with a Kd of greater than 1 µM), has low rotamase inhibitory activity (an apparent Ki of more than 2500 nM), binds with low affinity to calcineurin (requires concentrations greater than 30 µM to bind), or is non-immunosuppressive, as measured by the substantial absence of a drop in total blood lymphocyte counts in subjects to whom the agent is administered. NGPAs include, but are not limited to, non-FKBP12-binding ("non-binding") or low affinity FKBP-12 binding analogs of FK506; benzoquinone ansamycins, including geldanamycin, naturally occurring analogs of geldanamycin, including, but not limited to, herbimycin A and macbecin (DeBoer et al., *J. Antibiot*. (Tokyo) 23:442-447, 1970; Omura et al., *J. Antibiot.* (Tokyo) 32:255-261, 1979; Ono et al., *Gann*. 73:938-944, 1992), and structural analogs and derivatives thereof, such as geldampicin, tunicamycin, and dihydrogeldanamycin, as well as the compounds listed in Example 12; peptides, including an amino acid sequence of a particular polypeptide component of a steroid receptor complex at a site of interaction between that component and another component of the complex (such as the TPR domain), which interferes or competitvely binds with the component of the SRC; and antibodies that bind specifically to polypeptide components of steroid receptor complexes, e.g., anti-hsp-90, anti-FKBP-52, etc.) that interfere with the interaction of the bound polypeptide with another polypeptide in the steroid receptor complex. The neurotrophic agents include compounds that either physically disrupt association of the mature SRC (either by inhibiting association or promoting dissociation of the SRC), or inhibit interaction of components (such as p23, FKBP-52 or hsp-90) of the SRC.
**Neurotrophic:** Promoting nerve growth.
**Transformation:** conversion of the 9S non-DNA-binding form of a steroid receptor complex to the 4S DNA-binding form. The term "activation" refers to the conversion of a steroid receptor from a form that does not bind steroid ligand to a steroid-ligand-binding form.
**Rotamase Activity:** Rotamase (PPIase) activity can be determined, for example, as in WO 92/04370, and can be expressed as a K,. The *cis-trans* isomerization of the alanine-proline peptide bond in a model substrate, N-succinyl-Ala-Ala-Pro-Phe-4-nitroanalide, may be monitored spectrophotemetrically in a coupled assay with chymotrypsin, which releases 4-nitroanalide from the *trans* form of the substrate. The inhibitory effect upon the addition of different concentrations of inhibitor on the extent of the reaction is determined, and analysis of the change in the first order rate constant as a function of inhibitor concentration yields an estimate of the apparent Kᵢ. In the assay of the present invention, rotamase activity has been found to be unrelated to the neurotrophic activity of the compounds, and need not be determined.

"Known" or "recognized" compounds (such as FKBP-12 binding compounds or FK506 analogs) are those that have previously been reported in patents or publications, or that otherwise qualify as prior art.

### EXAMPLE 1

### Assays for Identifying Nerve Growth Promoting Agents

There are a number of well-known methods for assaying compounds that bind to hsp-90, FKBP-52, and other polypeptide components of a steroid receptor complex that can be used as an initial screen for candidate compounds that stimulate nerve regeneration. Compounds can subsequently be tested *in vitro* or *in vivo* for activity in stimulating nerve regeneration.

Examples include assays for the binding of a test compound to a polypeptide that is a component of a steroid receptor complex. An assay for detecting binding to hsp-90 is described, for example, by Whitesell et al. *(Proc. Natl. Acad. Sci. USA* 91:8324-8328, 1994). Commercial hsp-90 (StressGen Biotechnologies, Victoria, BC) dissolved in 20 µg/mL of TNESV buffer (50 mM Tris-HCl, pH 7.4/1% Nonidet P-40/2 mM EDTA/100 mM NaCl/1 mM orthovanadate/1 mM phenylmethylsulfonyl fluoride/20 µg leupeptin per mL/20 µg of aprotinin per ml) and the test compound are incubated for 45 min at 4°C with geldanamycin immobilized on a conventional solid support, e.g., geldanamycin-coupled agarose beads (Whitesell et al., *Proc. Natl. Acad. Sci. USA* 91:8324-8328, 1994). The beads are then washed with TNESV buffer and bound hsp-90 is eluted by heating in reducing loading buffer, and can be analyzed by SDS/PAGE and silver staining (Bio-Rad). Alternatively, if the hsp-90 is labeled, the assay can be performed for the bound label instead of the free label. Test compounds that compete with geldanamycin for binding to hsp-90 inhibit the binding of solubilized hsp-90 to the beads.

Similar assays can be performed to identify compounds that bind other steroid receptor complex polypeptide components. Binding to FKBP-52 can be assayed using recombinant FKBP-52 (Peattie et al., *Proc. Natl. Acad. Sci. USA* 89:10974-10978, 1992). Binding to p23 can be assayed using recombinant human p23 (Johnson et al., *Mol. Cell. Biol*. 14:1956-1963, 1994) and immobilized hsp-90. Purified hsp70 and recombinant p60 (Dittmar et al., *J. Biol. Chem*. 271:12833-12839, 1996) are also available for use in such binding assays.

Immunoassays can also be performed using conventional immunoassay methodologies and antibodies that are specific for steroid receptor complex components, e.g., antibodies against FKBP-52 (Tai et al., *Biochem*. 25:5269-5275, 1986), hsp-90 (Sanchez et al., *J. Biol. Chem*. 260:12398-12401, 1985; Catelli et al., *EMBO J.* 4:3131-3135, 1985; Schuh et al., *J. Biol. Chem.* 260:14292-14296, 1985), hsp70 (a serum that also recognizes hsp-90)(Erhart et al., *Oncogene* 3:595-603, 1988), and p23 (Johnson et al., *Mol. Cell. Biol*. 14:1956-1963, 1994).

A well-accepted qualitative assay for receptor transformation, which involves dissociation of hsp-90 from the receptor complex, is conversion of a receptor complex to a state that binds polyanions such as phosphocellulose (Kalimi et al., *J. Biol. Chem.* 250:1080-1086, 1975; Atger and Milgrom, *Biochem*. 15:4298-4304, 1976), ATP-Sepharose (Toft et al., *J. Steroid Biochem.* 7:1053-1059, 1976; Miller and Toft, *Biochem*. 17:173-177, 1978), and carboxymethol-Sephadex (Milgrom et al., *Biochem.* 12:5198-5205, 1973; Parchman and Litwack, *Arch. Biochem. Biophys*. 183:374-382, 1977).

An *in vitro* assay for nerve cell growth (neurite outgrowth) is provided in Example 2, and in Gold et al., *Exp. Neurol*. 147:269-278, 1997. *In vivo* assays for nerve regeneration are discussed in, for example, Gold et al., *Restor. Neurol. Neurosci.* 6:287-296, 1994; Gold et al., *J. Neurosci.* 15:7505-7516, 1995; Wang et al., *J. Pharmacol. Exp. Therapeutics* 282:1084-1093, 1997; Gold et al., *Exp. Neurol*. 147:269-278, 1997 and Gold et al., *Soc. Neurosci. Abst*. 23:1131, 1997, which examine the effects of systemic administration of a test compound on nerve regeneration and functional recovery following a crush injury to the rat sciatic nerve. The sciatic nerves of anaesthetized rats are exposed, and the nerves crushed using forceps at the level of the hip. Following the sciatic nerve crush, the test compound is administered to the rats, e.g., by subcutaneous injection or oral administration. Functional recovery is assessed by determining the number of days following nerve crush until the animal demonstrates onset of an ability to right its foot and move its toes, and the number of days until the animal demonstrates an ability to walk on its hind feet and toes.

Nerve regeneration is also assessed by sampling tissues from the sciatic nerve at known (0.5 cm) distances from the crush site and counting the number of myelinated fibers by light microscopy. The size of axons is calculated by electron microscopy. Axonal areas of both myelinated and unmyelinated fibers are determined by tracing the axolemma using a digitizing tablet connected to a computer with appropriate software. Cumulative histograms are constructed from these data and mean values and standard errors are calculated to assess the effect of administration of the test compound on axonal areas.

### EXAMPLE 2

### FK506 and Geldanamycin Promote Nerve Regeneration by a Common Mechanism

This Example illustrates that FK506 and geldanamycin promote nerve regeneration by a common mechanism. SH-SY5Y human neuroblastoma cells were maintained in DMEM medium (GIBCO) supplemented with 10% fetal calf serum (SIGMA). 50 IU/mL penicillin, and 50 mg/mL streptomycin (GIBCO) at 37°C in 7% CO₂. Cells were plated in six-well plates at 1 x 10⁶ cells/well and treated with 0.4 mM aphidicolin (SIGMA). At five days, cells were washed, treated with nerve growth factor (NGF) (Boehringer Mannheim, Indianapolis, IN) at 10 ng/mL (to induce process outgrowth) in the presence or absence of FK506 (1 and 10 nM) (Calbiochem-Novabiochem Int'l., La Jolla, CA) and/or geldanamycin (0.1, 1, and 10 nM) (Calbiochem-Novabiochem, La Jolla, CA) dissolved in the DMEM medium. Media was changed at 96 hours and replaced with fresh media containing the compounds (NGF plus FK506 and/or geldanamycin) for an additional 72 hours (total time, 168 hours). The top 50% of axonal lengths were selected for statistical analysis. All experiments were run in duplicate wells and repeated at least twice for reproducibility.

For analysis of neurite process length of cells, 20 fields per well were randomly photographed at 72 and 168 hours. Neurite lengths were measured on photographic prints using a Houston Instrument HI-PAD digitizing tablet connected to an IBM XT computer with appropriate software (Bioquant IV, R&M Biometrics, Nashville, TN); only those processes greater than two times the cell body length were measured. Data from identically treated wells were not different and were therefore combined. Mean values and histograms were constructed from these data. Histograms were compared using a Mann-Whitney U test, which makes no assumptions about the shape of the distribution.

The mean lengths of neuritic processes of untreated and treated cells are shown in Table 1:

**TABLE 1**

| **Mean Length of Top 50% of Neuritic Processes 168 Hours After Treatment with Geldanamycin in the Presence of NGF** | | |
|---|---|---|
| **Treatment** | **Mean Length (µM)** | **S.E.M.** |
| Untreated | 83.22 | 2.50 |
| NGF ( 10 ng/mL) | 107.98 | 4.52 |
| Geldanamycin (1 nM) + NGF (10 ng/mL) | 128.00 | 4.72 |
| Geldanamycin (10 nM) + NGF (10 ng/mL) | 109.62 | 4.20 |
| FK506 (10 nM) + NGF ( 10 ng/mL) | 155.64 | 5.40 |
| Geldanamycin (1 nM) + FK506 (10 nM) + NGF (10 ng/mL) | 145.26 | 4.02 |
| Geldanamycin (10 nM) + FK506 (10 nM) + NGF (10 ng/mL) | 134.82 | 3.34 |

Geldanamycin and FK506 each stimulate neurite outgrowth in a concentration dependent manner. The similar neurotrophic effects of geldanamycin and FK506, their additive effects at very low concentrations (e.g. 0.1 nM; data not shown), and their inhibitory effects at high concentrations (like high concentrations of either compound alone) demonstrate that the two compounds act on nerve cells to promote nerve outgrowth by a common mechanism. As the following examples will illustrate, that mechanism has now been found to involve an interaction of both compounds with components of the steroid receptor complex. FKBP12 does not appear to have a role in the stimulation of neurite outgrowth by either geldanamycin or FK506.

Call lines other than the SH-SY5Y human neuroblastoma cells can be used in the nerve growth assays. Examples of suitable other cell lines include PC-12 (rat pheochromocytoma), LA-N-5 cells (human neuroblastoma cells less differentiated than SY5Y cells), and Neuro-2a and NS20Y cells (mouse neuroblastoma).

### EXAMPLE 3

### FKBP-12 Knockout Mice Demonstrate FKBP-12 Not Involved in Neurotrophic Activity

FKBP-12 knockout mice (Shou. et al., *Nature* 391:489, 1998) were used to test whether FKBP-12 is necessary for FK506's ability to increase nerve elongation. Such mice usually die from severe cardiomyopathy between embryonic day 14.5 (E14.5) and birth, consistent with the known association between FKBP-12 and calcium release channels. No gross pathology has been noted in brains of these mice. Primary neuronal hippocampal cultures were prepared from E18.5 homozygote FKBP-12 knockout and wild-type mice. No difference was found in FK506's regenerative-promoting response of neurons in FKBP-12 knockout and wild-type mice. Mean axonal lengths of hippocampal neurons were not significantly different between FKBP-12 knockout and wild-type mice in drug-free cell cultures (203 ± 9.5 and 219 ± 8.0, respectively; mean ± S.E.M.) [two-way ANOVA and Scheffe's test of least significant differences; p = 0.68, df = 230)] or FK506-treated cultures (264 ± 18.2 and 276 ± 11.1, respectively) [two-way ANOVA and Scheffe's test of least significant differences; p = 0.94, df = 112)].

FK506 elicited a similarly significant increase over non-treated values in cells from FKBP-12 knockout [two-way ANOVA and Scheffe's test of least significant differences; p < 0.006, df = 144] and wild-type mice (two-way ANOVA and Scheffe's test of least significant differences; p < 0.002, df = 198]; i.e., 30% and 26%, respectively. Thus, neuronal cells from FKBP-12 knockout mice retain their responsiveness to the neurite outgrowth-promoting property of FK506. FKBP-12 is therefore not required for FK506 to promote neurite outgrowth *in vitro.*

### EXAMPLE 4

### FKBP-52 Blocks FK506 Neurotrophic Activity

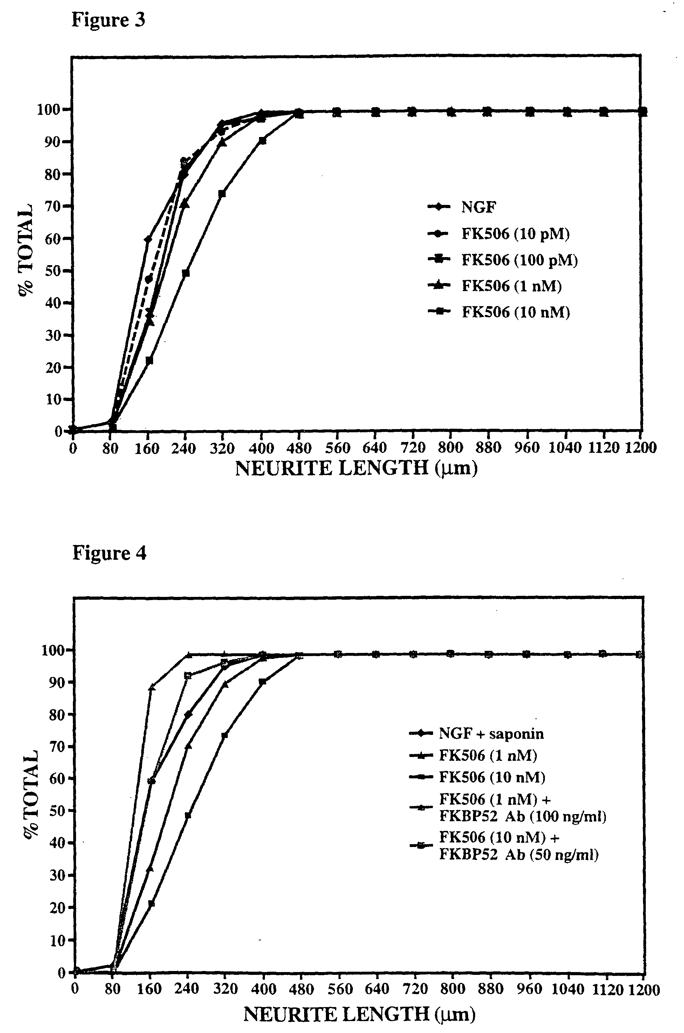

Neuroblastoma SH-SY5Y cells were used to examine human neurite *outgrowth in vitro* and to explore which neuroimmunophilin mediates the effect. SH-SY5Y cells do not extend processes in the absence of exogenous nerve growth factor (NGF), with optimal neurotrophic activity at 10 ng/ml NGF. Initial studies showed that FK506 increases neurite outgrowth in SH-SY5Y cells in a concentration-dependent manner, as shown in FIG. 3. Cumulative histograms of neurite lengths show that 10 pM - 10 nM FK506 significantly [Mann-Whitney U test (a= 0.05)] increases neurite outgrowth. However, 100 nM was less effective and, at 1000 nM or greater concentrations, neurite outgrowth was inhibited.

The involvement of FKBP-52 was demonstrated using a mouse monoclonal antibody, FKBP-52 Ab (StressGen Biotechnologies Corp., British Columbia, Canada) that does not interact with FKBP-12. To get the antibody into the cells, SH-SY5Y cells were permeabilized with saponin (30 µg/µl) for 10 min in the presence of the antibody; preliminary experiments showed that saponin treatment did not alter the response of the cells to NGF alone. As shown in FIG. 4, the FKBP-52 antibody significantly [Mann-Whitney U test (α = 0.05)] blocked the ability of FK506 (1 and 10 nM) to promote neurite outgrowth from SH-SY5Y cells in a concentration-dependent manner between 50 and 100 nM. Cumulative histograms of neurite lengths show that 100 nM FKBP-52 antibody completely blocks the action of FK506 at these concentrations. Surprisingly, the antibody blocked not only the effect of FK506 but also NGF's effect, suggesting a convergence of their signal transduction pathways perhaps involving the MAP kinase pathway (ERK2). Regardless of the underlying mechanism involved, FK506's neurite outgrowth-promoting property is totally dependent on its interaction with the immunophilin FKBP-52.

### EXAMPLE 5

### FKBP-52 Antibody Promotes Neurite Outgrowth

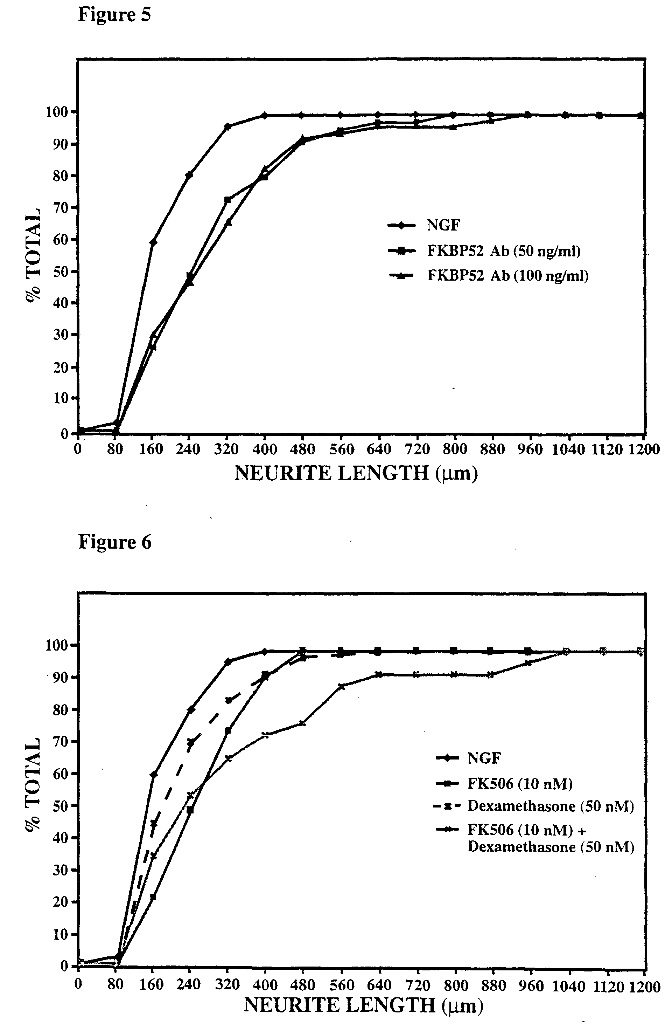

The FKBP-52 antibody (FKBP-52 Ab) itself possesses agonistic properties on neurite outgrowth. Cumulative histograms of neurite lengths in FIG. 5 show that FKBP-52 Ab significantly [Mann-Whitney U test (α = 0.05)] shifted the distribution of neurite lengths to the right in a concentration-dependent manner, indicating longer processes. In fact, the FKBP-52 Ab elicited even longer neurites per unit time than those maximally observed with FK506 (10 nM), producing some of the fastest growing neurites found to date (maximal length, 880 m). These findings demonstrate that it is possible to develop compounds which can distinguish between FKBP-52 and FKBP-12 (i.e., do not substantially bind to both immunophilins) while maintaining the ability to increase neurite outgrowth. This finding enables the development of a new class of neuroimmunophilin ligands: neuroimmunophilin compounds. having low or absent FKBP-12 binding affinity, which specifically bind to components of the SRC, and increase nerve regeneration by interacting selectively with FKBP-52 (or other components of the SRC, such as p23 or hsp-90)

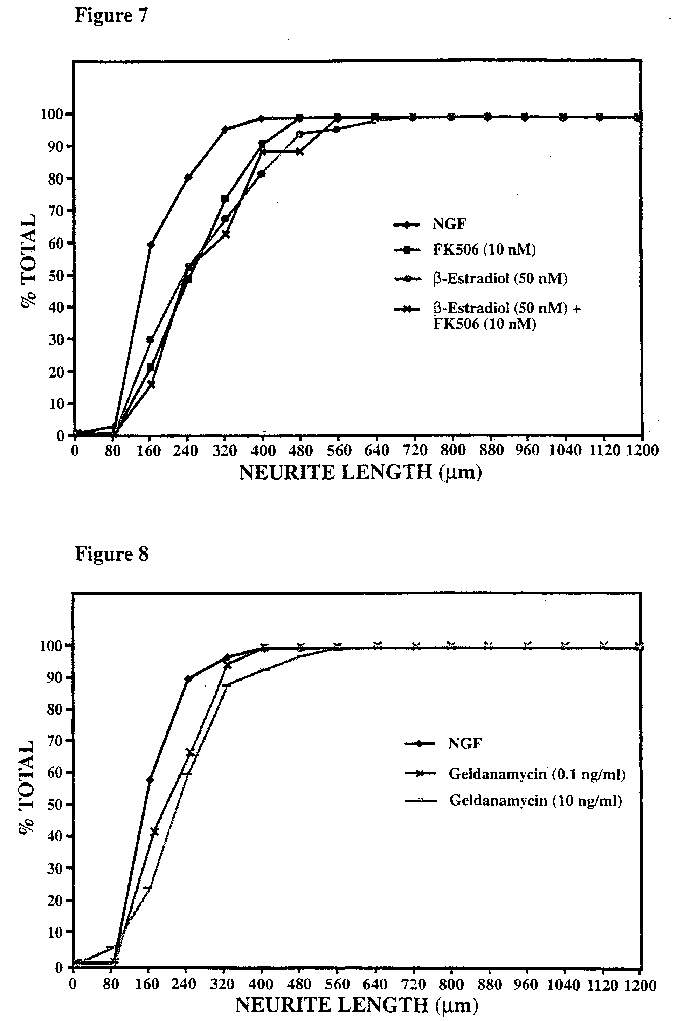

The synthetic glucocorticoid dexamethasone (FIG. 6) and β-estradiol (FIG. 7) both significantly increased neurite outgrowth in SH-SY5Y cells in a concentration-dependent manner. β-Estradiol (50 nM) produced a significantly [Mann-Whitney U test (α = 0.05)] greater positive effect on neurite outgrowth than dexamethasone (50 nM), suggesting a greater involvement of the estrogen receptor complex in SH-SY5Y cells. This is supported by the data also shown in FIG. 7 that the combination of β-estradiol and FK506 did not produce a further significant [Mann-Whitney U test (α = 0.05)] increase in neurite outgrowth, suggesting that these compounds act at the same steroid receptor sub-type. In contrast, the combination of dexamethasone and FK506 produced neurites (maximal length, 960 µm) that grew at least as, if not more, rapidly than those under FKBP-52 antibody modulation (FIG. 5), indicating that dexamethasone and FK506 act at different steroid receptor sub-types. Taken together, the finding that maximal neurite outgrowth elicited by FK506 and β-estradiol is not additive suggests the estrogen receptor complex plays a greater role than the glucocorticoid receptor complex in human SH-SY5Y neurite outgrowth promotion by FK506.

### EXAMPLE 6

### Geldanamycin Promotes Neurite Outgrowth by Disruption of Steroid Receptor Complex

SH-SY5Y cells were treated with geldanamycin, a benzoquinone antibiotic that blocks the reassociation of the mature steroid complex (containing FKBP-52 and p23), thereby preventing nuclear translocation and activation of steroid response elements. As shown in FIG. 8, geldanamycin (0.1 - 10 nM) alone significantly [Mann-Whitney U test (α = 0.05)] increased neurite outgrowth in a concentration-dependent fashion. Thus, disruption of the steroid receptor complex is sufficient to increase neurite outgrowth. Since geldanamycin and steroid hormones have opposite effects on the translocation of the steroid receptor ligand component to the nucleus, these results demonstrate that the promotional effect of these compounds on neurite outgrowth is mediated by a mechanism other than nuclear translocation of the steroid receptor ligand-binding component and activation of steroid response elements. Using this information, it is now possible to exploit the structure of geldanamycin to develop a new class of hsp-90-binding compounds for use in nerve regeneration.

Assays for determining disruption of the steroid receptor complex are set forth in Example 10.

### EXAMPLE 7

### Neurotrophic Effect of Geldanamycin Combined with Other Compounds

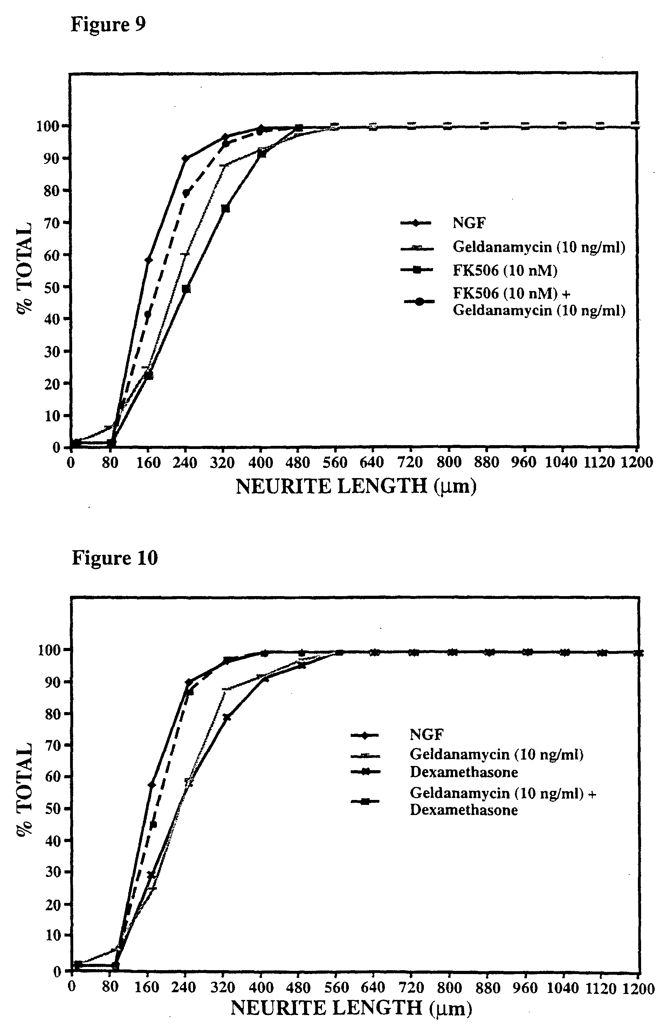

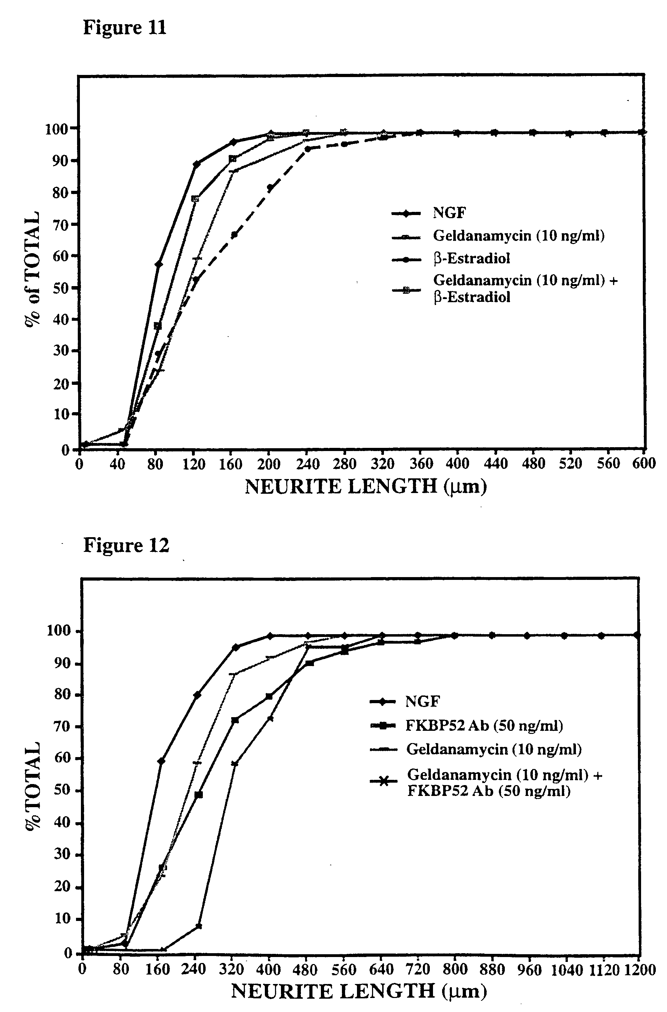

To explore the interaction of other neurotrophic substances with geldanamycin, SH-SY5Y cells were co-treated with 1 nM (not shown) or 10 nM geldanamycin and various other compounds (FIGS. 9-12). On the one hand, geldanamycin (10 nM) significantly [Mann-Whitney U test (α = 0.05)] inhibited neurite outgrowth promotion by FK506 (FIG. 9), dexamethasone (FIG. 10) or β-estradiol (FIG. 11); at 0.1 nM, geldanamycin was less effective in inhibiting the neurite outgrowth-promoting effect of all these compounds (not shown). On the other hand, geldanamycin (10 nM) significantly [Mann-Whitney U test (α = 0.05)] enhanced the neurite outgrowth-promoting effect of the FKBP-52 antibody (FIG. 12). The combined effect of FKBP-52 antibody and geldanamycin is consistent with their different binding sites on hsp-90; geldanamycin binds to the N-terminus and FKBP-52 to the C-terminus portions of hsp-90 (Scheigel et al., *J. Bio. Chem*. 272:8007, 1997). This finding shows an interaction at the steroid level complex for all tested compounds, yet reveals that the antibody acts somewhat differently.

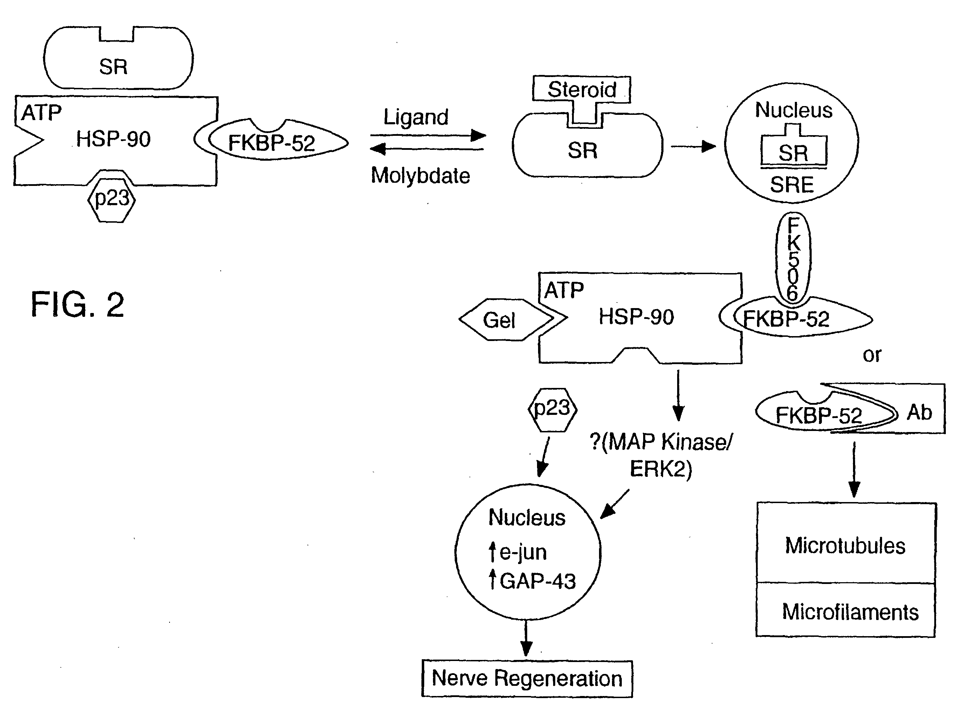

These findings can be explained by the model shown in FIG. 2 in which geldanamycin produces a conformational change (not dissociation) in hsp-90 which, via its adenosine triphosphatase activity, leads to an activation (adenosine diphosphate) state in which p23 dissociates from the complex. In contrast, this conformational change may be blocked, thereby preventing release of p23, when FKBP-52 is bound to FK506, because FK506 does not dissociate FKBP-52 from the complex; a similar interaction may occur in the presence of steroid hormones to prevent the conformational change in hsp-90. This model indicates that the FKBP-52 antibody dissociates FKBP-52 from the complex, perhaps by altering its degree of phosphorylation and thereby reducing its binding to hsp-90, and leads to a conformational change in hsp-90 that results in release of p23. Thus, the combination of geldanamycin and the FKBP-52 antibody are additive (not inhibitory) because dissociation of FKBP-52 from hsp-90 would not prevent the geldanamycin-induced conformational change that releases p23.

In addition, the association of FKBP-52 with microtubules (Czar et al., *Mol. Endocrinol* 8:1731, 1994) and perhaps microfilaments such as actin (Tai et al., *Biochem.* 32:8842, 1993) following its dissociation from hsp-90, would promote the greater neurite outgrowth seen with FKBP-52 antibody than with FK506. The microtubules are involved with neurite outgrowth and axonal elongation, hence association of FKBP-52 with those elements (after dissociation from the SRC) is a particularly effective mechanism of promoting neurite growth.

### EXAMPLE 8

### Stabilization of Steroid Receptor Complex Inhibits Neurite Outgrowth

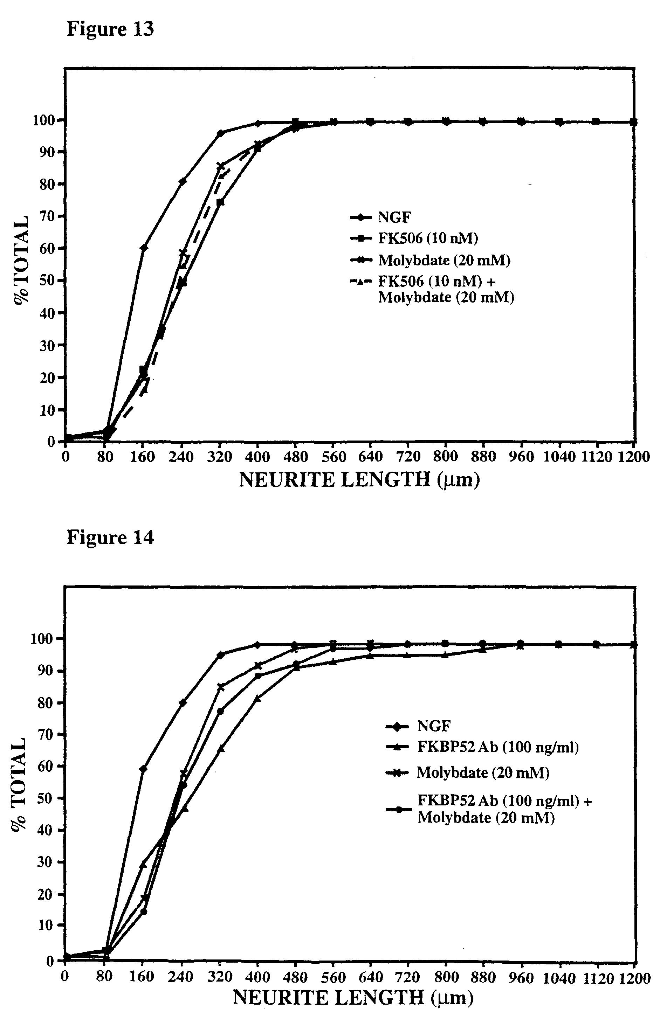

This Example shows that prevention of the dissociation of the steroid receptor complex inhibits neurite outgrowth, as predicted by the model shown in FIG. 2. SH-SY5Y cells were treated with sodium molybdate, a transition metal oxyanion, that at a concentration of 20 mM prevents dissociation of the complex in intact cells. Surprisingly, molybdate (20 mM) itself exhibited a modest but significant [Mann-Whitney U test (α = 0.05)] agonist effect on neurite outgrowth (FIG. 13). As predicted, molybdate (20 mM) reduced the neurite outgrowth promotion elicited by FK506, the distribution of neurite lengths produced by FK506 in the presence of molybdate being not significantly [Mann-Whitney U test (α = 0.05)] different from that with molybdate alone (FIG. 13). Furthermore, molybdate (20 mM) significantly [Mann-Whitney U test (α = 0.05)] inhibited the neurite outgrowth-promoting effects of FKBP-52 antibody (FIG. 14).

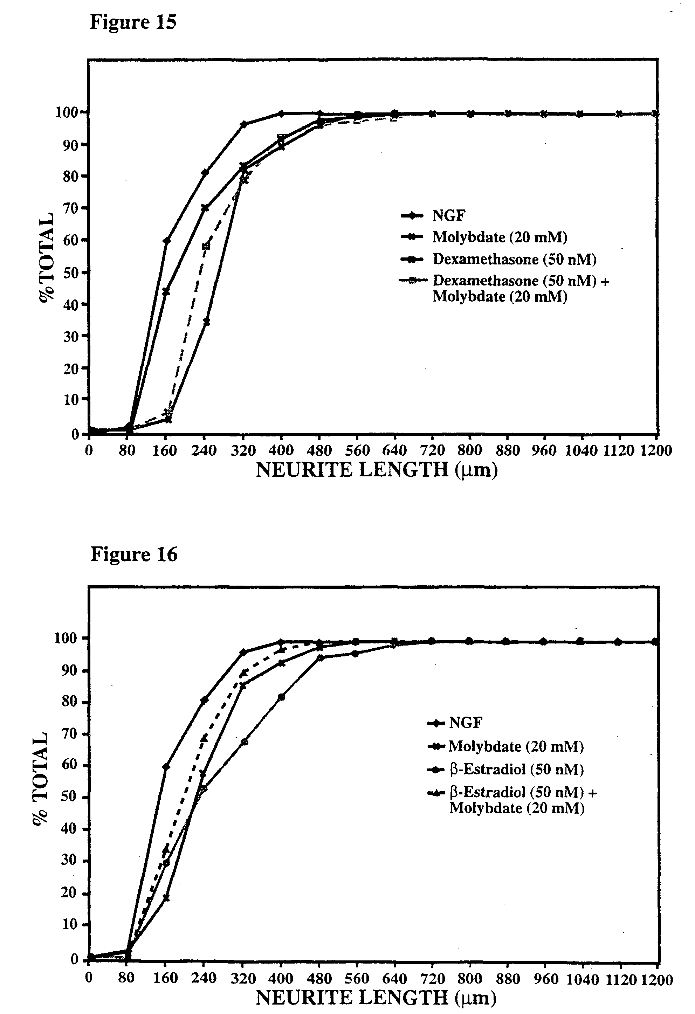

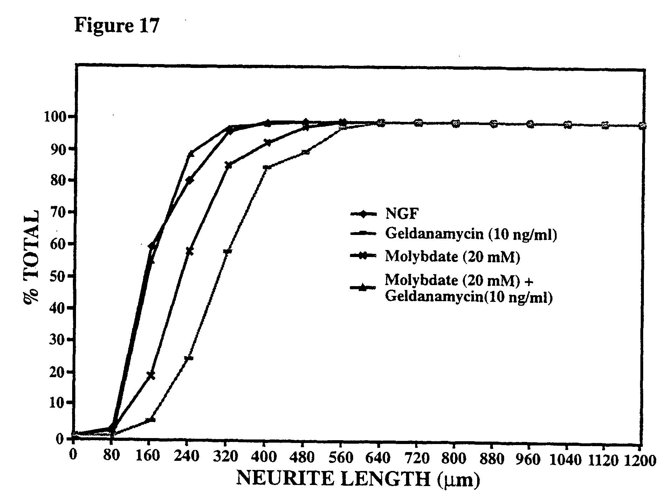

The neurite outgrowth-promoting effect of molybdate (20 mM) in the presence of dexamethasone was significantly [Mann-Whitney U test (α = 0.05)] reduced compared to molybdate alone (FIG. 15). Furthermore, molybdate (20 mM) completely [Mann-Whitney U test (α = 0.05)] inhibited the neurite outgrowth-promoting effect of β-estradiol (FIG. 16) and geldanamycin (FIG. 17); the larger degree of interaction between molybdate and β-estradiol compared to molybdate and dexamethasone is consistent with a greater involvement of the estrogen receptor complex in human SH-SY5Y neurite outgrowth. Molybdate produced similar but less marked effects at a lower (2 mM) concentration (not shown).

While it is unclear how molybdate alone increases outgrowth, the data (showing that molybdate inhibits the activity of all agents, including FKBP-52 antibody) indicate that dissociation of the receptor complex is an essential step for activation of the neurite development pathway. Prevention of this dissociation by molybdate inhibits the neurotrophic activity of geldanamycin and other neurotrophic agents that act by disrupting association of the complex. Hence inhibition of neurotrophic activity by adding molybdate to an assay can constitute a test for determining whether a neurotrophic agent is structurally or functionally disrupting the SRC.

### EXAMPLE 9

### Determination of Rotamase Inhibition Activity

Some embodiments of the use of the present invention have low or absent inhibition of peptidylprolyl isomerase (rotamase) activity. Inhibition of this activity can be evaluated by techniques known in the an, such as that described in U.S. Patent No. 5,614,547. Inhibition is expressed as an apparent Ki for *cis-trans* isomerization of an alanine-proline bond in a model substrate, N-succinyl-Ala-Ala-Pro-Phe-p-nitroanilide, which is monitored spectrophometrically in a chymotrypsin-coupled assay, which releases para-nitroanilide from the trans form of the substrate. The inhibition of this reaction caused by the addition of different concentrations of inhibitor is determined, and the data is analyzed as a change in the first-order rate constant as a function of inhibitor concentration to yield the apparent Ki values.

In a plastic cuvette are added 950 ml of ice cold assay buffer (25 mM HEPES, pH 7.8, 100nM NaCl), 10 ml of FKBP (2.5 mM in 10 mM Tris-Cl pH j7.5, 100 mM NaCl, 1 mM dithiothreitol), 25 ml of chymotrypsin (50 mg/ml in 1 mM HCl) and 10 ml of test compound at various concentrations in dimethyl sulfoxide. The reaction is initiated by the addition of 5 ml of substrate (succinyl-Ala-Phe-Pro-Phe-para-nitroanilide, 5 mg/ml in 2.35 mM LiCl in thfluroethanol).

Absorbance at 390 nm versus time is monitored for 90 seconds using a spectrophotometer and the rate constants are determined from the absorbance versus time data files. Inhibitors that have an apparent Ki of 2500 or greater, for example greater than 5000 or even 10,000, are considered to have "low" rotamase inhibition.

### EXAMPLE 10

### Assays for Determining if Agent Binds to and Disrupts SRC Function

Assays for detecting binding to components of the SRC are given in Example 1. Detection of disruption of the SRC can be assessed by additional assays.

Disruption of the SRC by release of p23 can be assessed by the techniques disclosed in Whitesell and Cook, 1996, where benzoquinone ansamycin binding to hsp-90 was shown to result in complete loss of p23 protein from glucocorticoid receptor immunoprecipitates, which was associated with a rapid, noncompetitive loss of dexamethasone binding activity, and a slower (2-8 hours) marked decline in the cellular level of glucocorticoid receptor protein. Using this approach, drug treatment did not disrupt coprecipitation of hsp-90 with glucocorticoid receptor, and a complete loss of detectable p23 from glucocorticoid receptor precipitates.

Whitesell and Cook performed affinity precipitation by lysing cells in TNESV buffer (50 mM Tris-HCl, pH 7.4%/1% Nonidet P-40/2 mM EDTA/100 mM NaCl/1mM orthovanadate/1 mM phenylmethysulfonyl fluoride/20 µg/ml leupeptin/20 µg/ml aprotinin) and lysates (0.75 mg of total protein per precipitation) incubated with geldanamycin-coupled beads. Bound proteins are eluted by heating in reduced loading buffer and analyzed by SDS-PAGE followed by Coomassie blue staining. Immnoprecipitation from cell lysates is performed using a specific monoclonal antibody BuGR-2, and protein G Sepharose beads (Pharmacia). For experiments involving coprecipitation of GR with heteroprotein complex components, cells are lysed in detergent-free hypotonic buffer with 10 mM sodium molbydate. Immunoblot detection of proteins in total cells lysates, geldanamycin affinity precipitates, and glucocorticoid receptor immunoprecipitates are performed after SDS-PAGE and electrophoretic transfer of proteins to nitrocellulose. BuGR-2 hybridoma supernatant (1:40) is used for detection of rodent derived GR while a peptide-derived rabbit polyclonal antibody (1:250; PA1-512, Affinity Bioreagents; Golden, CO) is used for the human GR. Hsp-90 and hsp-70 are detected with antibodies AC88 and N27F3-4 respectively (1:5000; StressGen; Victoria, BC, Canada). Ascites containing antibody JJ3 (1:1000) is used to blot for p23. Polyclonal rabbit anti-ubiquitin antiserum (1:500; Sigma Chemcial Co.) is used to detect ubiquitinated proteins after blots are autoclaved for 20 minutes to fully denature ubiquitinated proteins and enhance their detection. Detection is achieved using appropriate peroxidase-conjugated secondary antibodies (1:20,000) and chemiluminescent substrate (Kierkegaard and Perry Laboratories, Gaithersburg, MD).

Loss of dexamethasone binding activity can be determined with a binding assay in which HeLa cells (2 x 10⁵/well, 24-well plate) are treated with various concentrations of geldanamycin for varying periods of time in complete medium at 37 degrees C. At the end of the treatment interval, medium is aspirated and monolayers washed twice with ice-cold PBS containing 1 % BSA and 0.1% sodium azide (binding buffer). Monolayers are then incubated for 60 minutes on ice with 1.0 µCi/well (48 nM) [³H]dexamethasone (Amersham, 82 Ci/mmol) in binding buffer with or without 5mM non-radioactive dexamethasone. After this binding interval, wells are washed four times with cold binding buffer and then extracted with 0.5 ml ethanol for 30 minutes. The ethanol solution is transferred to scintillation vials and evaporated to dryness before standard liquid scintillation counting. Specific binding is calculated as the counts per minute bound in the absence of excess nonradioactive dexamethasone less the counts per minute bound in its presence. Similar measurements can be made with estrogen and other steroid receptors, to detect disruption in ligand binding, by substituting those steroids for dexamethasone.

A loss of detectable p23 (disappearance of the p23 band) from receptor immunoprecipitates can indicate loss of p23 in association with disruption of the SRC.

### EXAMPLE 11

### Preparation of Antibodies

The present disclosure also contemplates the preparation of antibodies against components of the SRC. The components of the SRC can be purified by techniques known in the art, such as immunoprecipitation. Monoclonal or polyclonal antibodies may be produced to either the SRC component proteins, peptide fragments, or mutant forms of these proteins. Optimally, antibodies raised against the protein will specifically detect the protein. That is, antibodies raised against the protein would recognize and bind the protein and would not substantially recognize or bind to other proteins found in human cells. The determination that an antibody specifically detects a protein is made by any one of a number of standard immunoassay methods; for instance, the Western blotting technique (Sambrook et al., 1989).

To determine that a given antibody preparation specifically detects the protein by Western blotting, total cellular protein is extracted from human cells (for example, lymphocytes) and electrophoresed on a sodium dodecyl sulfate-polyacrylamide gel. The proteins are then transferred to a membrane (for example, nitrocellulose) by Western blotting, and the antibody preparation is incubated with the membrane. After washing the membrane to remove non-specifically bound antibodies, the presence of specifically bound antibodies is detected by the use of an anti-mouse antibody conjugated to an enzyme such as alkaline phosphatase; application of the substrate 5-bromo-4-chloro-3-indolyl phosphate/nitro blue tetrazolium results in the production of a dense blue compound by immune-localized alkaline phosphatase.

Antibodies which specifically detect the protein will, by this technique, be shown to bind to the protein band (which will be localized at a given position on the gel determined by its molecular weight). Non-specific binding of the antibody to other proteins may occur and may be detectable as a weak signal on the Western blot. The non-specific nature of this binding will be recognized by one skilled in the an by the weak signal obtained on the Western blot relative to the strong primary signal arising from the specific protein binding.

Antibodies that specifically bind to a protein component of the SRC belong to a class of molecules that are referred to herein as "specific binding agents." Specific binding agents that are capable of specifically binding to the SRC protein may include polyclonal antibodies, monoclonal antibodies (including humanized monoclonal antibodies) and fragments of monoclonal antibodies such as Fab, F(ab')2 and Fv fragments, as well as any other agent capable of specifically binding to a protein component of the SRC.

Monoclonal antibody to epitopes of the SRC protein components identified and isolated as described can be prepared from murine hybridomas according to the classical method of Kohler and Milstein (1975) or derivative methods thereof. Briefly, a mouse is repetitively inoculated with a few micrograms of the selected protein over a period of a few weeks. The mouse is then sacrificed, and the antibody-producing cells of the spleen isolated. The spleen cells are fused by means of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall (1980), and derivative methods thereof. Selected positive clones can be expanded and their monoclonal antibody product harvested for use. Detailed procedures for monoclonal antibody production are described in Harlow and Lane (1988). In addition, protocols for producing humanized forms of monoclonal antibodies (for therapeutic applications) and fragments of monoclonal antibodies are known in the art.

Polyclonal antiserum containing antibodies to heterogeneous epitopes of a single protein can be prepared by immunizing suitable animals with the expressed protein, which can be unmodified or modified to enhance immunogenicity. Effective polyclonal antibody production is affected by many factors related both to the antigen and the host species. For example, small molecules tend to be less immunogenic than others and may require the use of carriers and adjuvant. Also, host animals vary in response to site of inoculations and dose, with both inadequate or excessive doses of antigen resulting in low titer antisera. Small doses (ng level) of antigen administered at multiple intradermal sites appears to be most reliable. An effective immunization protocol for rabbits can be found in Vaitukaitis et al. (1971).

Booster injections can be given at regular intervals, and antiserum harvested when antibody titer thereof, as determined semi-quantitatively, for example, by double immunodiffusion in agar against known concentrations of the antigen, begins to fall. See, for example, Ouchterlony et al. (1973). Plateau concentration of antibody is usually in the range of 0.1 to 0.2 mg/ml of serum (about 12 µM). Affinity of the antisera for the antigen is determined by preparing competitive binding curves, as described, for example, by Fisher (1980).

A third approach to raising antibodies against the SRC proteins is to use synthetic peptides synthesized on a commercially available peptide synthesizer based upon the predicted amino acid sequence of the protein components of the SRC.

### EXAMPLE 12

### Geldanamycin Analogs and Derivatives

The term "geldanamycin derivative" refers to compounds that are structurally analogous to geldanamycin in their ability to stimulate neurite outgrowth. Geldanamycin consists of a closed ansa ring with a planar benzoquinone embedded in it.

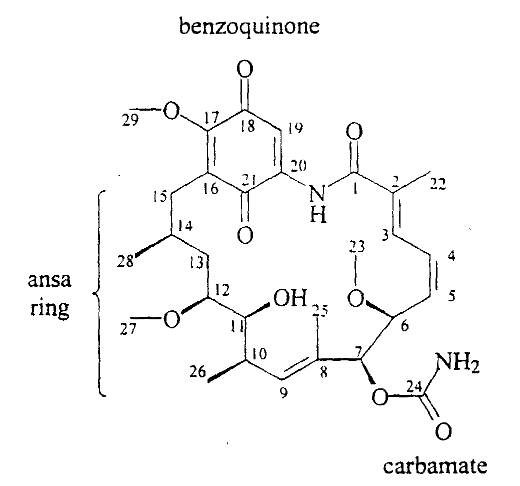

The ansa ring is sterically hindered because its backbone includes a planar amide and three carbon-carbon double bonds (two of them arranged in a 1.3-diene), and nine of its sixteen backbone atoms carry nonhydrogen substitutents such as a carbonyl, a carbamate (-OC(O)NH₂), a hydroxyl, two methoxy and four methyl groups. The crystal structure of geldanamycin has been set forth, and the structure/activity relationships of the benzoquinone ansamycins have been described in Stebbins et al., *Cell* 89:239-250, 1997; Schnur et al., *J. Med. Chem*. 38:3813-3820, 1995; and Schnur et al., *J. Med. Chem*. 38:3806-3812, 1995. Geldanamycin derivatives may have the carbamate group and ansa ring of geldanamycin (Schur et al., *J. Med. Chem*. 38:3806-3812, 1995), and/or have modifications at functional groups such as the C23 methoxy and C22 methyl groups (Stebbins et al., *Cell* 89:239-250, 1997). Geldanamycin derivatives are also discussed in U.S. Patent No. 5,3877,584, 4,261,989, and 3,987,035, and in Japanese Patent Applications 88041885, 56100766, and 89002593, for example.

The structures of some of the benzoquinone ansamycins that are structural analogs of geldanamycin are set forth in Table 2.

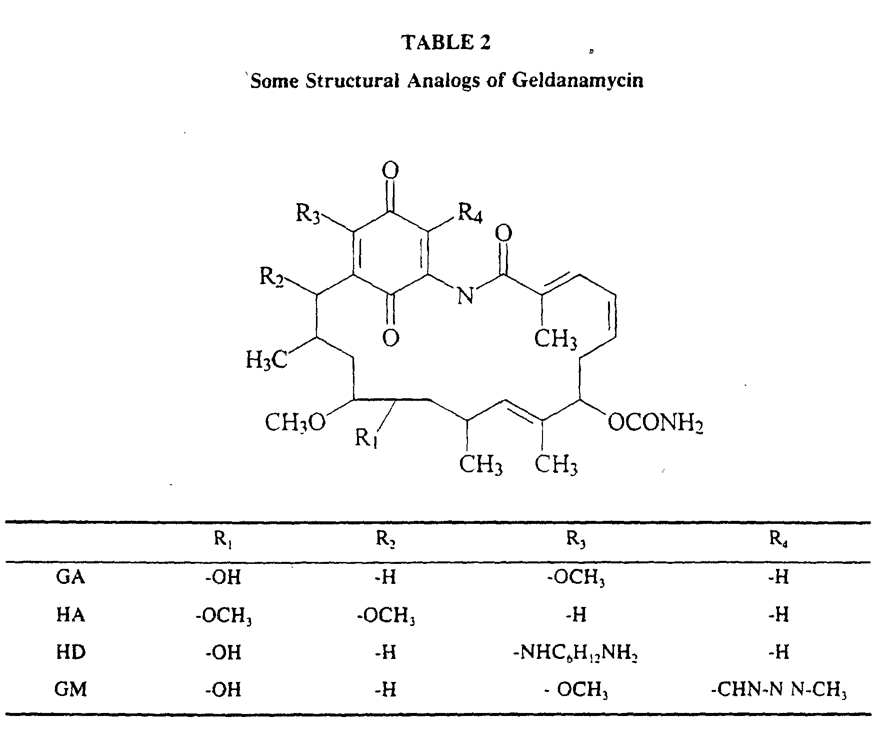

Additional benzoquinone ansamycin analogs of geldanamycin are shown in Tables 3 and 4. Table 3 illustrates several synthesis schemes for gledanamycin derivatives, while Table 4 sets forth substitutions for the derivatives. as described more fully in Schnur, et al., *J. Med. Chem.* 38:3813-3820, 1995.

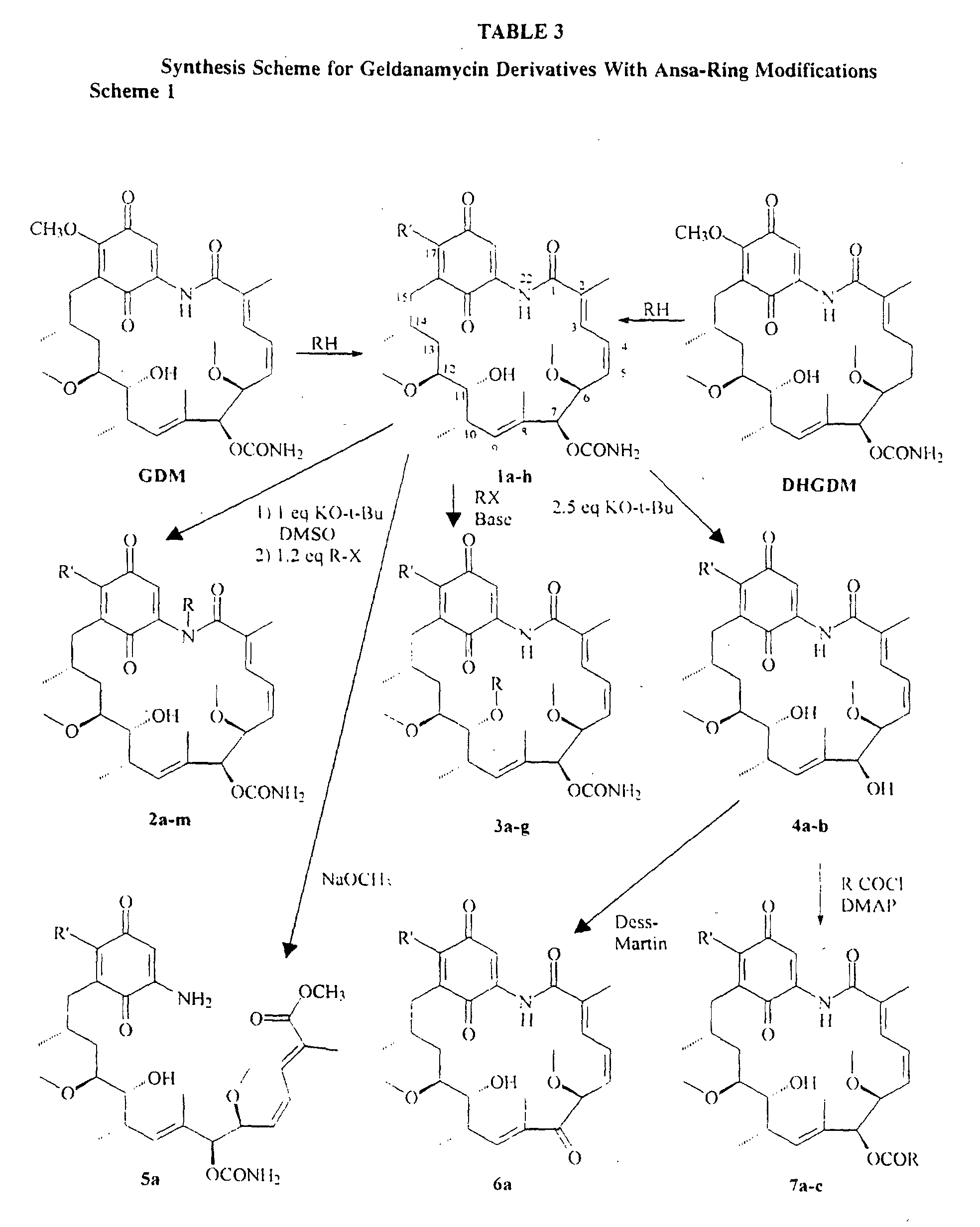

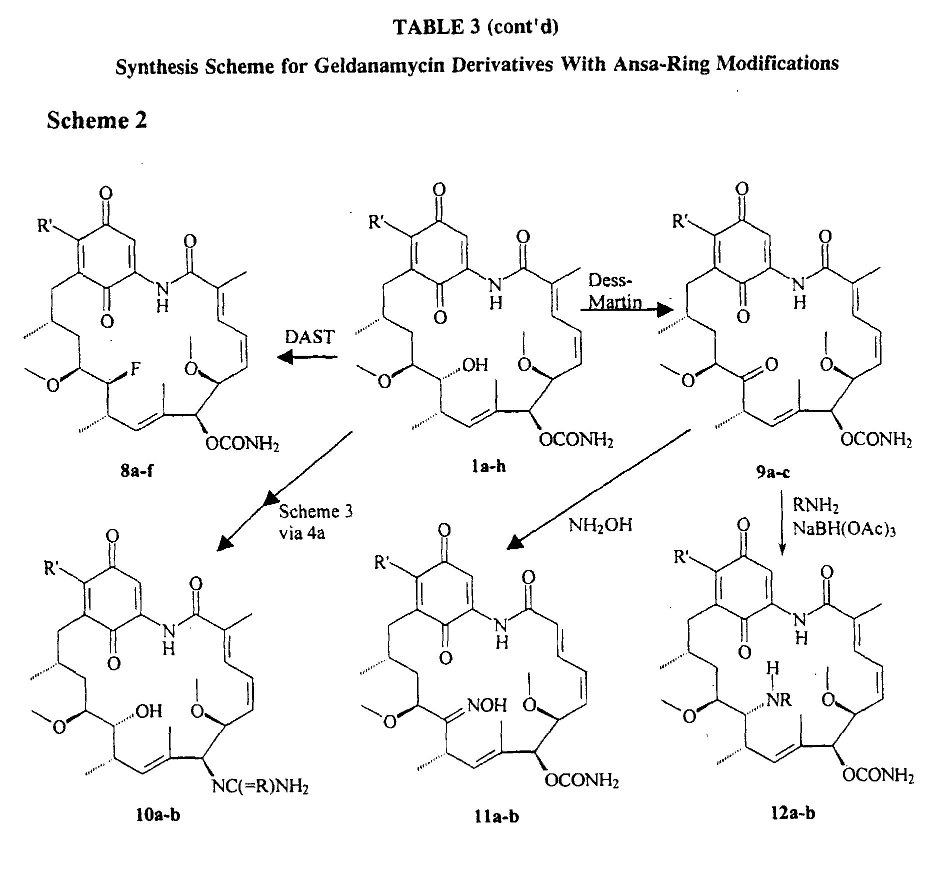

**TABLE 4**

| **Substitutions for Compounds Shown in Table 3** | | |
|---|---|---|
| compd^{*a*} | R | R' |
| HBM A | | |
| GDM | | OCH₃ |
| DHGDM | | OCH₃ |
| 1a | | -N(CH₂)₃- |
| 1b | | NH₂ |
| 1c | | NHCH₂CH=CH₂ |
| 1d | | NHCH(CN₃)₂ |
| 1e | | NHCH₂ |
| 1f* | | -N(CH₂)₃- |
| 1g* | | NH₂ |
| 1h* | | NHCH₂CH=CH₂ |
| 2a | phenacyl | NH₂ |
| 2b | 3',4'-dichlorophenacyl | NH₂ |
| 2c | 3'-iodo-4'-azidophenacyl | NH₂ |
| 2d | 2'-methoxyphenacyl | NH₂ |
| 2c* | 2'-methoxyphenacyl | NH₂ |
| 2f | 4'-methoxyphenacyl | NH₂ |
| 2g | 4'-nitrophenacyl | NH₂ |
| 2h | 1'-napthacyl | NH₂ |
| 2i | 2'-napthacyl | NH₂ |
| 2j | 4'-azidophenacyl | NH₂ |
| 2k | 4'-azidophenacyl | -N(CH₂)₃- |
| 2l | 2'-oxopropyl | NH₂ |
| 2m | 2'-pyridylmethyl | NH₂ |
| 3a | COCH₈ | -N(CH₂)₃- |
| 3b | CONHSO₂NHCH(CH₃)₂ | -N(CH₂)₃- |
| 3c | CONHSO₂N[(CH₂)₂]₂NCH₃ | -N(CH₂)₃- |
| 3d | CONH₂ | -N(CH₂)₃- |
| 3e | CONHSO₂N(CH₂)₃ | NHCH₂CH=CH₂ |
| 3f | CONHSO₂NHCH(CH₃)₂ | NHCH₂CH=CH₂ |
| 3g | CONHSO₂N(CH₂)₂NCH₃ | NHCH₂CH=CH₂ |
| 4a | | -N(CH₂)₃- |
| 4b | | NH₂ |
| 5a | | -N(CH₂)₃- |
| 6a | | NHCH(CH₂)₃ |
| 7a | COCO₂H | -N(CH₂)₃- |
| 7b | CONHCH₂CH₃ | NHCH₂CH=CH₂ |
| 7c | COCH₂NH₂ | NHCH₂CH=CH₂ |
| 8a | | -N(CH₂)₃- |
| 8b* | | -N(CH₂)₃- |
| 8c | | NHCH₂CH=CH₂ |
| 8d* | | NHCH₂CH=CH₂ |
| 8e | | NH₂ |
| 8f | | NHCH(CH₂)₂ |
| 9a | | NHCH₂CH=CH₂ |
| 9b | | -N(CH₂)₃- |
| 9c | | NHCH₃ |
| 10a | S | -N(CH₂)₃- |
| 10b | O | -N(CH₂)₃- |
| 11a | | -N(CH₂)₃- |
| 11b | | NHCH(CH₃)₂ |
| 12a | CH₂CH=CH₂ | NHCH₃ |
| 12b | CH₂C₆H₅ | NHCH₃ |

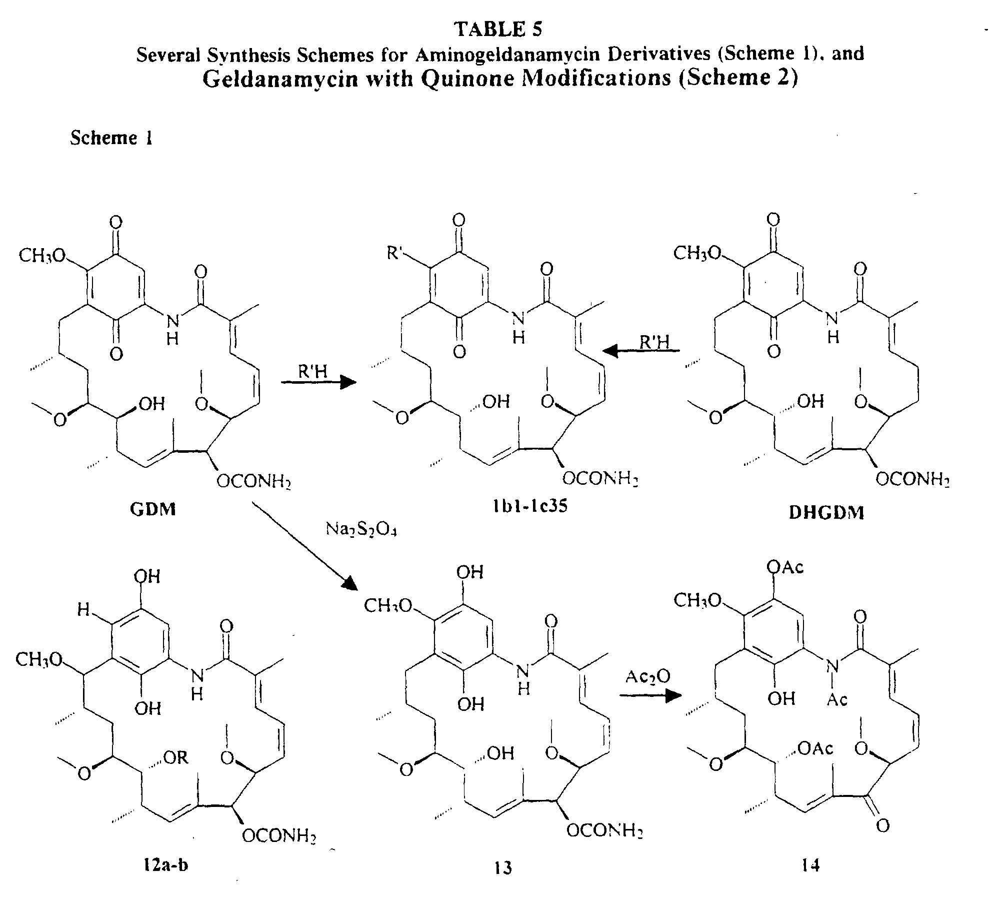

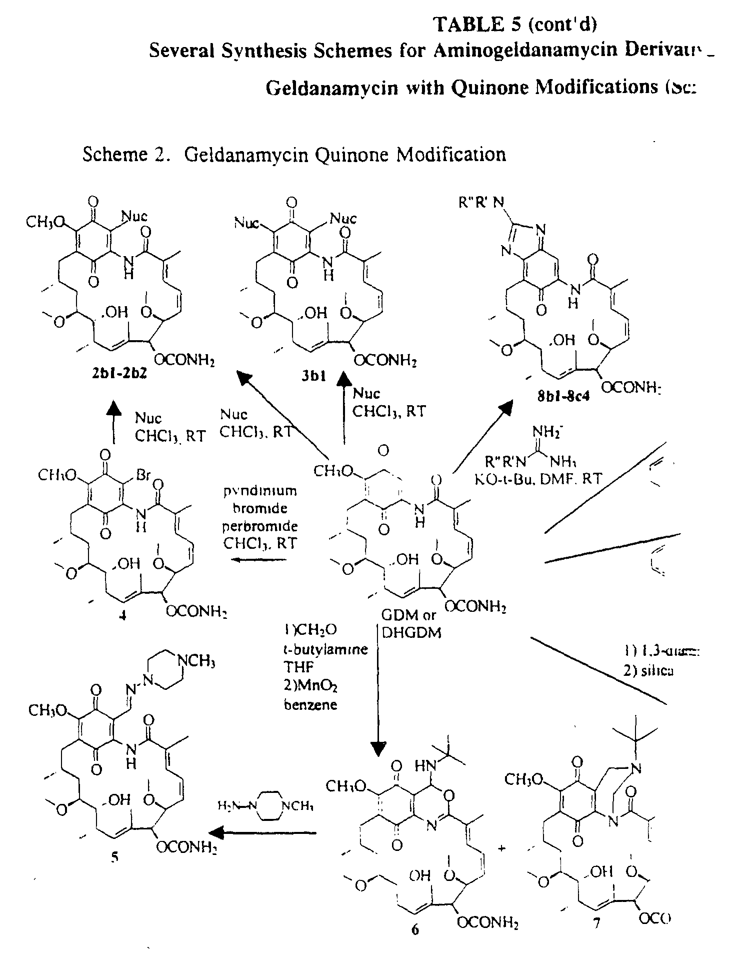

Some substitution patterns for the derivatives in Table 5 are shown in Table 6.

**TABLE 6**

| **Some Substitutions for Compounds Shown in Table 5** | | |
|---|---|---|
| compd^{*a*} | R | R' |
| HBM A | | |
| GDM | | OCH₃ |
| DHGDM | | OCH₃ |
| 1a | | -N(CH₂)₃- |
| 1b | | NH₂ |
| 1c | | NHCH₂CH=CH₂ |
| 1d | | NHCH(CH₃)₂ |
| 1e | | NHCH₂ |
| 1f* | | -N(CH₂)₃- |
| 1g* | | NH₂ |
| 1h* | | NHCH₂CH=CH₂ |
| 2a | phenacyl | NH₂ |
| 2b | 3',4'-dichlorophenacyl | NH₂ |
| 2c | 3'-iodo-4'-azidophenacyl | NH₂ |
| 2d | 2'-methoxyphenacyl | NH₂ |
| 2e* | 2'-methoxyphenacyl | NH₂ |
| 2f | 4'-methoxyphenacyl | NH₂ |
| 2g | 4'-nitrophenacyl | NH₂ |
| 2h | 1'-napthacyl | NH₂ |
| 2i | 2'-napthacyl | NH₂ |
| 2j | 4'-azidophenacyl | NH₂ |
| 2k | 4'-azidophenacyl | -N(CH₂)₃- |
| 2l | 2'-oxopropyl | NH₂ |
| 2m | 2'-pyridylmethyl | NH₂ |
| 3a | COCH₈ | -N(CH₂)₃- |
| 3b | CONHSO₂NHCH(CH₃)₂ | -N(CH₂)₃- |
| 3c | CONHSO₂N[(CH₂)₂]₂NCH₂ | -N(CH₂)₃- |
| 3d | CONH₂ | -N(CH₂)₃- |
| 3e | CONHSO₂N(CH₂)₃ | NHCH,CH =CH, |
| 3f | CONHSO₂NHCH(CH₃)₂ | NHCH₂CH=CH₂ |
| 3g | CONHSO₂N(CH₂)₂NCH₃ | NHCH₂CH=CH₂ |
| 4a | | -N(CH₂)₃- |
| 4b | | NH, |
| 5a | | -N(CH₂)₃- |
| 6a | | NHCH(CH₂)₃ |
| 7a | COCO,H | -N(CH₂)₃- |
| 7b | CONHCH₂CH₃ | NHCH₂CH=CH₂ |
| 7c | COCH₂NH₂ | NHCH₂CH=CH₂ |
| 8a | | -N(CH₂)₃- |
| 8b* | | -N(CH₂)₃- |
| 8c | | NHCH₂CH=CH₃ |
| 8d* | | NHCH₂CH=CH₂ |
| 8e | | NH₂ |
| 8f | | NHCH(CH₂)₂ |
| 9a | | NHCH₂CH=CH₂ |
| 9b | | -N(CH₂)₃- |
| 9c | | NHCH₃ |
| 10a | S | -N(CH₂)₃- |
| 10b | O | -N(CH₂)₃- |
| 11a | | -N(CH₂)₃- |

| | | |
|---|---|---|
| 11b | | NHCH(CH₃)₂ |
| 12a | CH₂CH=CH₂ | NHCH₃ |
| 12b | CH₂C₆H₅ | NHCH₃ |

Analogs can also be modified by appending appropriate functionalities by well-known methods to enhance selected biological properties, including increasing penetration of the analogs into a given cellular compartment (e.g., blood, lymphatic system, central nervous system, etc.), increase oral availability, increase solubility to permit administration by injection, alter metabolism, and alter rate of excretion.

In some particular embodiments, analogs have a molecular weight below about 750 atomic mass units (a.m.u.) (as the parent compound, although the salts of such compounds can have higher molecular weights).

### EXAMPLE 13

### FK506 and Rapamycin Analogs

The term "FK506 analogs" refers to compounds that are structurally analogous to FK506 in their ability to stimulate neuritic outgrowth. Some FK506 analogs, such as V-10,367, retain the FKBP12 binding domain but lack the structural components of the effector domain that interacts with calcineurin. The FK506 analogs may bind FKBP12 with low or high affinity. V-10,367, for example, binds FKBP12 with high affinity (K_{d}< 1 nM) (Armistead et al., *Acta Crystallogr*. 51:522-528, 1995).

There has been an intense effort to design compounds that are structurally related to FK506 and that share the ability of FK506 to inhibit FKBP12 and thereby cause immunosuppression. See, for example: Bierer et al., *Science* 250:556-559, 1990; Van Duyne et al., *Science* 252:839-842, 1991; Van Duyne et al., *J. Mol. Biol.* 229:105-124, 1993; Hauske et al., *J. Med. Chem*. 35:4284-4296, 1992; Holt et al., *J. Am. Chem. Soc.* 115:9925-9938, 1993; Holt et al., *Bioorg. Med. Chem. Lett.* 3:1977-1980, 1993: Teague and Stocks, *Bioorg. Med. Chem. Lett.* 3:1947-1950, 1993; Wang et al., *Bioorg. Med. Chem. Lett.* 4:1161-1166, 1994; Yamashita et al., *Bioorg, Med. Chem. Lett*. 4:325-328, 1994; Stocks et al., *Bioorg. Med. Chem. Lett*. 4:1457-1460, 1994; Goulet et al., *Perspect. Drug Disc. Design* 2:145-162, 1994; Wilson et al., *Acta Cryst.* D51:511-521, 1995; Armistead et al., *Acta Cryst*. D51:522-528, 1995; U.S. Patents No. 5, 192,773, 5,330,993, 5,516,797, 5,612,350, 5,614,547, 5,622,970, 5,654,332; and published international patent applications WO 92/00278, WO 92/04370, WO 92/19593, WO 92/21313, WO 94/07858, and WO 96/40633. These references set forth the structure of FK506 and some of its known analogs, such as V-10,367, which lacks the effector domain (shown in brackets) that inhibits calcineurin. A "known" or "recognized" compound is a compound (such as an FK506 analog) that has previously been reported in patents or publications that qualify as prior art.

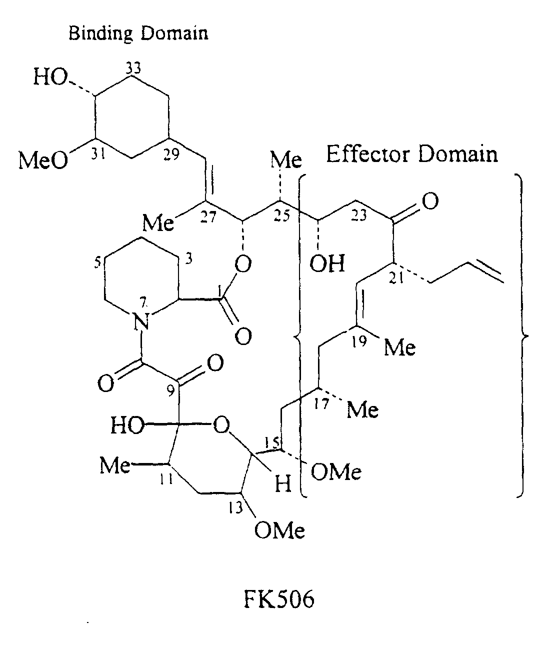

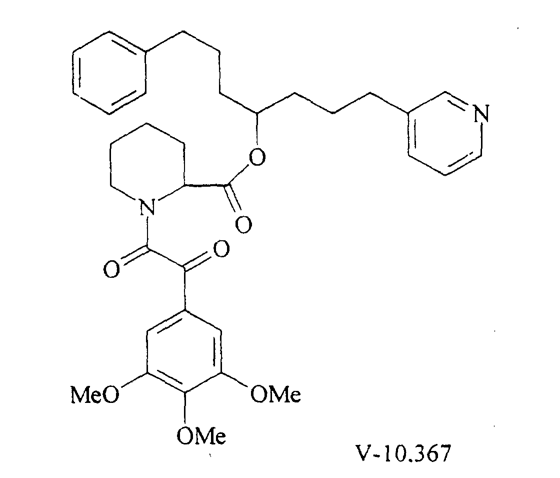

FK506 analogs have a wide range of binding affinities for FKBP-12. The mechanism for neurotrophic activity of FK506 presented herein indicates that the effectiveness of FK506 and FK506 analogs in stimulating nerve cell growth is unrelated to their ability to bind FKBP-12. Instead, their effectiveness in stimulating nerve cell growth relates to ability of such compounds to physically or functionally disrupt the steroid receptor complex, for example by interfering with the interaction of FKBP-52 and hsp-90 in a steroid receptor complex, or by promoting dissociation of p23 from the complex.

A "non-binding FK506 analog" is defined as an FK506 analog that does not substantially bind to FKBP-12. An FK506 analog with low affinity for binding FKBP-12 refers to an analog that binds FKBP12 with an apparent K_{d} of greater than 10 µM as measured using well-known assays, and preferably greater than 30 µM, and more preferably greater than 100 µM. Values for the apparent K_{d} can be determined, for example, by a competitive LH-20 binding assay performed as described, for example, in Harding et al., *Nature* 341:758-760. 1989 (using 32-[1-¹⁴C]-benzoyl FK506 as a reporting ligand; Siekierka et al., *Nature* 341:755-757, 1989, using [³H]dihydro-FK506 as a reporting ligand); and U.S. Patent No. 5,654,332.

Alternatively or additionally, the analog may be one that does not significantly inhibit FKBP-12 rotamase activity when administered to a patient at dosage levels of about 0.01 to about 100 mg/kg body weight/day. Assays for inhibition of FKBP12 rotamase activity are described in Example 9.

Non-binding FK506 analogs are non-immunosuppressive, as can be demonstrated by well-known assays, e.g., as discussed in U.S. Patent No. 5,516,797, WO 92/21313, WO 92/19593, and WO 92/04370.

"Rapamycin analogs" include compounds structurally similar to rapamycin, for example WAY-124,466 shown in Ocain et al., *Biochem. Biophys. Res. Commun.* 192-1340-1346, 1993. This analog is identical to rapamycin, except that it has been modified in the triene region, and has a Ki for PPlase activity of 12.5 nM, as determined by the methods shown in that reference. It is also non-immunosuppressive, as determined in that reference by an inability to inhibit the proliferation of murine thymocytes. Other rapamycin analogs include other macrocyclic trienes, mono- and diacylated derivatives esterified at the 31 and 42 positions (U.S. Patent No. 4,313,885) and water soluble prodrugs of rapamycin (U.S. Patent No. 4,650,803); hydrogenated derivatives in which one, two, or three of the double bonds at the 1-, 3-, or 5-positions have been reduced to the corresponding alkane, or a pharmaceutically acceptable salt thereof (U.S. Patent No. 5,023,262); oxidized derivatives wherein a hydroxyl group (such as the group at the 42-position) has been oxidized to the corresponding ketone (for example 42-oxorapamycin or a pharmaceutically acceptable salt, as described in U.S. Patent No. 5,023,263); 7,29-bisdesmethyl rapamycin (U.S. Patent No. 5,093,338); and rapamycin cleaved between the C-31 and C-32 carbons due to base degradation by a reverse aldol reaction (U.S. Patent No. 5,138,051).

### EXAMPLE 14

### Methods of Use

The neurotrophic compounds are administered in an effective amount sufficient to stimulate nerve growth or regeneration compared to a control. Suitable local concentrations for nerve cell growth or nerve regeneration can be readily assessed using an *in vitro* assay, e.g., the assay described in Example 1. Alternatively, nerve cell growth or regeneration can be determined by an *in vivo* assay, or by direct or indirect signs of nerve cell growth and regeneration in a subject (for example a restoration of motor and/or sensory function in the hand in the area of innervation of a previously transected median nerve). Preferably, the increase in nerve cell growth or regeneration rate is at least 10%, preferably at least 30%, and most preferably 50% or more compared to a control. Preferred dosage levels are between about 0.1 to about 400 mg/kg per day of the FK506 analog for subcutaneous delivery. For oral administration, dosage level examples are between about 0.01 to about 40 mg/kg/day. Alternatively, the dose can be sufficient to achieve tissue concentrations that have been shown to be neurotrophic *in vitro*.

Pharmaceutical compositions can be periodically administered to a mammalian subject (e.g., a human patient), in need of such treatment, to promote neuronal regeneration and functional recovery and to stimulate neurite outgrowth and thereby to treat various neuropathological states, including damage to peripheral nerves and the central nervous system caused by physical injury (e.g., spinal cord injury and trauma, sciatic or facial nerve lesion or injury, limb transplantation following amputation), disease (e.g., diabetic neuropathy), cancer chemotherapy (e.g., neuropathy induced by acrylamide, taxol, vinca alkaloids and doxorubicin), brain damage associated with stroke and ischemia, and neurological disorders including, but not limited to, various peripheral neuropathic and neurological disorders related to neurodegeneration including, but not limited to: trigeminal neuralgia, glossopharyngeal neuralgia, Bell's palsy, myasthenia gravis, muscular dystrophy, amyotrophic lateral sclerosis, progressive muscular atrophy, progressive bulbar inherited muscular atrophy, herniated, ruptured or prolapsed vertebral disk syndromes, cervical spondylosis, plexus disorders, thoracic outlet destruction syndromes, peripheral neuropathies such as those caused by lead, acrylamides, gamma-diketones (glue-sniffer's neuropathy), carbon disulfide, dapsone, ticks, porphyria, Gullain-Barré syndrome, Alzheimer's disease, Parkinson's disease, and Huntington's chorea.

In addition, pharmaceutical compositions according to the present invention display a wide range of other therapeutic or prophylactic properties, including, treatment of stroke (see, e.g., Sharkey and Butcher, *Nature* 371:336-339, 1994, Vagita et al., *Life Sciences* 59:1643-1650, 1996; Tokime et al., *Neurosci. Lett*. 206:81-84, 1996; Drake et al., *Acta. Physiol. Scand.* 158:155-159, 1996; and Kuroda et al., *Neurosci. Res. Comm*. 19:83-90, 1996), AIDS dementia (see, e.g., Dawson and Dawson, *Adv. Neuroimmunol*. 4:167-173, 1994: and Sekigawa et al., *J. Clin. Immunol.* 15:312-317, 1995); hair growth (Yamamoto et al., *J. Investig. Dermatol.* 102:160-164, 1994; Jiang et al., *J. Investig. Dermatol*. 104:523-525, 1995); and connective tissue disorders (see e.g., Steinmann et al., *J. Biol. Chem.* 266:1299-1303, 1991), and as a male contraceptive (see e.g., Hisatomi et al., *Toxicology* 109:75-83, 1996).

A transection of a peripheral nerve or a spinal cord injury can be treated by administering a nerve growth stimulating amount of the agent to the mammal and grafting to the peripheral nerve or spinal cord a nerve graft such as an allograft (Osawa et al., *J. Neurocytol*. 19:833-849, 1990; Buttemeyer et al., *Ann. Plastic Surgery* 35:396-401, 1995) or an artificial nerve graft (Madison and Archibald, *Exp. Neurol.* 128:266-275. 1994; Wells et al., *Exp. Neurol.* 146:395-402, 1997). The space between the transected ends of the peripheral nerve or spinal cord is preferably filled with a non-cellular gap-filling material such as collagen, methyl cellulose, etc., or cell suspensions that promote nerve cell growth, such as Schwann cells (Xu et al., *J. Neurocytol*. 26:1-16, 1997), olfactory cells, and sheathing cells (Li et al. *Science* 277:2000-2002, 1997). The nerve growth promoting agent can be included together with such cellular or non-cellular gap-filling materials, or administered systemically before, during or after the nerve graft procedure.

### EXAMPLE 15

### Pharmaceutical Formulations

Pharmaceutical formulations according to the present disclosure encompass formulations that include an amount (for example, a unit dosage) of the neurotrophic agent together with one or more non-toxic pharmaceutically acceptable excipients, including carriers, diluents, and/or adjuvants, and optionally other biologically active ingredients. Standard pharmaceutical formulation techniques are used, such as those disclosed in Remington's Pharmaceutical Sciences, Mack Publishing Co., Easton, PA (19th Edition).

A pharmaceutical formulation according to the disclosure includes one or more of the neurotrophic agents of the present invention, and can also include, for example, one or more other biologically active ingredients, such as FK506 or an FKBP12-binding FK506 analog, NGF, IGF-1, FGF, FGF. PDGF, BDNF, CNTF, GDNF, NT-3, and NT 4/5. When the SRC disrupting agents are combined with a second neurotrophic agent, the two agents are ideally selected such that they structurally or functionally disrupt the SRC by acting at different SRC components. For example, the first agent may be geldanamycin (which promotes dissociation of p23) and the second agent may be FKBP52-Ab (which interferes with association of FKBP-52 to hsp-90). In particular embodiments, the composition includes NGF, or another agent that stimulates nerve growth in combination with the SRC disrupting complex, or the neurotrophic action of which is augmented by administration in combination with the SRC disrupting agent.

The dosage of the combined biologically active agents is sufficient to achieve tissue concentrations at the site of action that are similar to those that are shown to achieve in vivo nerve regeneration. Pharmaceutical formulations may include, for example, an amount of a NGF, such that the subject receives a dosage of between about 0.01 to 100 µg/kg body weight/day. The NGF (or other adjuvant) can be administered separately, concurrently, consecutively, or within less than about five hours of each other.

The compositions can be in the form of tablets, capsules, powders, granules, lozenges, liquid or gel preparations, such as oral, topical, or sterile parenteral solutions or suspensions (e.g., eye or ear drops, throat or nasal sprays, etc.), transdermal patches, and other forms known in the art.

Such pharmaceutical compositions can be administered systemically or locally in any manner appropriate to the treatment of a given condition, including orally, parenterally, rectally, nasally, buccally, vaginally, topically, optically, by inhalation spray, or via an implanted reservoir. The term "parenterally" as used herein includes, but is not limited to subcutaneous, intravenous, intramuscular, intrasternal, intrasynovial, intrathecal, intrahepatic, intralesional, and intracranial administration, for example, by injection or infusion. For treatment of the central nervous system, the pharmaceutical compositions preferably readily penetrate the blood-brain barrier when peripherally administered or are administered intraventricularly.

Pharmaceutically acceptable carriers include, but are not limited to, ion exchangers, alumina, aluminum stearate, lecithin, serum proteins (such as human serum albumin), buffers (such as phosphates), glycine, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol, and wool fat.

Tablets and capsules for oral administration can be in a form suitable for unit dose presentation and can contain conventional pharmaceutically acceptable excipients. Examples of these include binding agents such as syrup, acacia, gelatin. sorbitol, tragacanth. and polyvinylpyrrolidone; fillers such as lactose, sugar, corn starch, calcium phosphate, sorbitol, or glycine; tableting lubricants, such as magnesium stearate, talc, polyethylene glycol, or silica; disintegrants, such as potato starch: and dispersing or wetting agents, such as sodium lauryl sulfate. Oral liquid preparations can be in the form of, for example, aqueous or oily suspensions, solutions, emulsions, syrups or elixirs, or can be presented as a dry product for reconstitution with water or other suitable vehicle before use.

The pharmaceutical compositions can also be administered parenterally in a sterile aqueous or oleaginous medium. The composition can be dissolved or suspended in a non-toxic parenterally-acceptable diluent or solvent, e.g., as a solution in 1,3-butanediol. Commonly used vehicles and solvents include water, physiological saline, Hank's solution, Ringer's solution, and sterile, fixed oils, including synthetic mono- or di-glycerides, etc. For topical application, the drug may be made up into a solution, suspension, cream, lotion, or ointment in a suitable aqueous or non-aqueous vehicle. Additives may also be included, e.g., buffers such as sodium metabisulphite or disodium edeate; preservatives such as bactericidal and fungicidal agents, including phenyl mercuric acetate or nitrate, benzalkonium chloride or chlorhexidine, and thickening agents, such as hypromellose.

The dosage unit involved depends, for example, on the condition treated, nature of the formulation, nature of the condition, embodiment of the claimed pharmaceutical compositions, mode of administration, and condition and weight of the patient. Dosage levels are typically sufficient to achieve a tissue concentration at the site of action that is at least the same as a concentrations that has been shown to be neurotrophic *in vitro*. For example, a dosage of about 0.1 to about 400 mg/kg per day of the active ingredient may be useful in the treatment of the conditions listed above.

The compounds can be used in the form of salts preferably derived from inorganic or organic acids and bases, including, but not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include, but are not limited to, ammonium salts, alkali metal salts (such as sodium and potassium salts), alkaline earth metal salts (such as calcium and magnesium salts), salts with organic bases (such as dicyclohexylamine salts), N-methyl-D-glucamine, and salts with amino acids (such as arginine, lysine, etc.). Basic nitrogen-containing groups can be quaternized, e.g., with such agents as lower alkyl halides (such as methyl, ethyl, propyl, and butyl chlorides, bromides, and iodides), dialkyl sulfates (such as dimethyl, diethyl, dibutyl, an diamyl sulfates), long-chain halides (such as decyl, lauryl, myristyl, and stearyl chlorides, bromides, and iodides), aralkyl halides (such as benzyl and phenethyl bromides), etc. Water or oil-soluble or dispersible products are produced thereby.

Pharmaceutical compositions can be included in a kit accompanied by instructions for intended use, for example instructions required by a pharmaceutical regulatory agency, such as the Food and Drug Administration in the United States.

### Summary

The foregoing examples illustrate that neurotrophic properties of neuroimmunophilin ligands (FK506) and steroid hormones are mediated by physical or functional disruption of steroid receptor complexes. Some of the components of the complex that can act as targets for disruption include FKBP-52, hsp-90 and p23, which are all present together in mature steroid receptor complexes, which can be disrupted by geldanamycin. Since FKBP-52 can associate with microtubules and dynein, and via its TPR motifs also associate with kinesin, it can also have a direct role in the movement (axonal transport) of cytoskeletal elements and, consequently, axonal elongation.

## Claims

1. Use of a composition that disrupts assembly of a steroid receptor complex for the manufacture of a medicament for the stimulation of nerve growth, wherein the composition comprises a compound that is
(a) an FK506 structural analog which binds to FKBP-12 with a K_{d} of greater than 1 µM;
(b) a benzoquinone ansamycin
(c) a rapamycin structured analog which binds to FKBP-12 with a K_{d} of greater than 1µ M.
(d) a peptide comprising one or more tetratricopeptide repeat domains;
(e) an antibody to one or more components of the steroid receptor complex.

2. The use of claim 1, wherein the composition comprises an additional neurotropic factor other than one of the factors of claim 1.

3. The use of claim 2, wherein the additional neurotropic factor is a nerve growth factor.

4. The use of any one of claims 1-3, wherein the compound is an FK506 structural analog which binds to FKBP-12 with a K_{d} of greater than 1 µM.

5. The use of any one of claims 1-3, wherein the compound is an FK506 structural analog which binds to FKBP-12 with a K_{d} of greater than 10 µM.

6. The use of any one of claims 1-3, wherein the compound is a bonzoquinone ansamycin.

7. The use of claim 6, wherein the benxoquinone ansamycin is geldanamycin.

8. The use of any one of claims 1-3, wherein the compound is a peptide comprising one or more tetratricopeptide repeat domains.

9. The use of any one of claims 1-3, wherein the compound is an antibody to one or more components of the steroid receptor complex.

10. A method of screening for a compound that stimulates nerve cell growth, comprising
determining if a test compound disrupts assembly or function of a steroid receptor complex by causing p23, FKBP-52, or hsp-90 disassociation from the steroid receptor complex, inhibiting p23, FKBP-52, or hsp-90 association with the steroid receptor complex, or inhibiting the interaction of p23, FKBP-52, or hsp-90 with the steroid receptor complex; and
selecting a compound that disrupts assembly or function of the steroid receptor complex, thereby selecting a compound that stimulates nerve growth.

11. The method of claim 10, further comprising testing the selected compound in an additional assay to measure neurite outgrowth induced by the compound

12. The method of claim 10, wherein determining if a compound disrupts assembly of the steroid receptor complex comprises determining if the compound causes p23, FKBP-52, or hsp-90 disassociation from the steroid receptor complex.

13. The method of claim 12, wherein determining if a compound disrupts assembly of the steroid receptor complex comprises determining if the compound causes p23 disassociation from the steroid receptor complex.

14. The method of claim 12, wherein determining if a compound disrupts assembly of the steroid receptor complex comprises determining if the compound causes FKBP-52 disassociation from the steroid receptor complex.

15. The method of claim 12, wherein determining if a compound disrupts assembly of the steroid receptor complex comprises determining if the compound causes hsp-90 disassociation from the steroid receptor complex.

16. The method of claim 10, wherein determining if a compound disrupts assembly of the steroid receptor complex comprises determining if the compound inhibits p23, FKBP-52, or hsp-90 association with the steroid receptor complex.

17. The method of claim 16, wherein determining if a compound disrupts assembly of the steroid receptor complex comprises determining if the compound inhibits p23 association with the steroid receptor complex.

18. The method of claim 16, wherein determining if a compound disrupts assembly of the steroid receptor complex comprises determining if the compound inhibits FKBP-52 association with the steroid receptor complex.

19. The method of claim 16, wherein determining if a compound disrupts assembly of the steroid receptor complex comprises determining if the compound inhibits hsp-90 association with the steroid receptor complex.

20. The method of claim 10, wherein determining if a compound disrupts assembly of the steroid receptor complex comprises determining if the compound inhibits the interaction of p23, FKBP-52, or hsp-90 with the steroid receptor complex.

21. The method of claim 20, wherein determining if a compound disrupts assembly of the steroid receptor complex comprises determining if the compound inhibits the interaction of p23 with the steroid receptor complex.

22. The method of claim 20, wherein determining if a compound disrupts assembly of the steroid receptor complex comprises determining if the compound inhibits the interaction of FKBP-52 with the steroid receptor complex.

23. The method of claim 20, wherein determining if a compound disrupts assembly of the steroid receptor complex comprises determining if the compound inhibits the interaction of hsp-90 with the steroid receptor complex.

24. The method of claim 10, wherein determining if a compound disrupts assembly of the steroid receptor complex comprises determining whether the compound inhibits association or promotes dissociation of p23 from hsp90.

25. The method of claims 10, wherein determining if a compound disrupts assembly or function of the steroid receptor complex comprises determining whether the compound binds to a geldanamycin binding site.

26. The method of claim 10, wherein determining if a compound disrupts assembly or function of the steroid receptor complex comprises determining whether the compound inhibits association or promotes dissociation of FKBP-52 from hsp-90.

## Patentansprüche

1. Verwendung einer Zusammensetzung, die den Aufbau eines Steroidrezeptorkomplexes stört, zur Herstellung eines Arzneimittels für die Stimulierung von Nervenwachstum, wobei die Zusammensetzung eine Verbindung umfaßt, die
(a) ein FK506-Strukturanalog, das sich an FKBP-12 bindet, mit einer K_{d} von mehr als 1 µM;
(b) ein Benzochinonansamycin;
(c) ein Rapamycin-Strukturanalog, das sich an FKBP-12 bindet, mit einer K_{d} von mehr als 1 µM;
(d) ein Peptid, das eine oder mehrere Tetratricopeptid-Wiederholungsdomänen umfaßt;
(e) ein Antikörper zu einer oder mehreren Komponenten des Steroidrezeptorkomplexes
ist.

2. Verwendung nach Anspruch 1, **dadurch gekennzeichnet, daß** die Zusammensetzung einen zusätzlichen anderen neurotropen Faktor als einen der Faktoren von Anspruch 1 umfaßt.

3. Verwendung nach Anspruch 2, **dadurch gekennzeichnet, daß** der zusätzliche neurotrope Faktor ein Nervenwachstumsfaktor ist.

4. Verwendung nach einem der Ansprüche 1 bis 3, **dadurch gekennzeichnet, daß** die Verbindung ein FK506-Strukturanalog ist, das sich an FKBP-12 bindet, mit einer K_{d} von mehr als 1 µM.

5. Verwendung nach einem der Ansprüche 1 bis 3, **dadurch gekennzeichnet, daß** die Verbindung ein FK506-Strukturanalog ist, das sich an FKBP-12 bindet, mit einer K_{d} von mehr als 10 µM.

6. Verwendung nach einem der Ansprüche 1 bis 3, **dadurch gekennzeichnet, daß** die Verbindung ein Benzochinonansamycin ist.

7. Verwendung nach Anspruch 6, **dadurch gekennzeichnet, daß** das Benzochinonansamycin Geldanamycin ist.

8. Verwendung nach einem der Ansprüche 1 bis 3, **dadurch gekennzeichnet, daß** die Verbindung ein Peptid ist, das eine oder mehrere Tetratricopeptid-Wiederholungsdomänen umfaßt.

9. Verwendung nach einem der Ansprüche 1 bis 3, **dadurch gekennzeichnet, daß** die Verbindung ein Antikörper zu einer oder mehreren Komponenten des Steroidrezeptorkomplexes ist.

10. Verfahren zum Screenen auf eine Verbindung, die Nervenzellwachstum stimuliert, welches umfaßt,
daß bestimmt wird, ob eine Testverbindung den Aufbau oder die Funktion eines Steroidrezeptorkomplexes stört, indem p23-, FKBP-52- oder hsp-90-Disassoziation vom Steroidrezeptorkomplex bewirkt wird, p23-, FKBP-52- oder hsp-90-Assoziation mit dem Steroidrezeptorkomplex gehemmt wird oder die Wechselwirkung von p23, FKBP-52 oder hsp-90 mit dem Steroidrezeptorkomplex gehemmt wird; und
daß eine Verbindung ausgewählt wird, die den Aufbau oder die Funktion des Steroidrezeptorkomplexes stört, wodurch eine Verbindung ausgewählt wird, die Nervenwachstum stimuliert.

11. Verfahren nach Anspruch 10, welches weiter umfaßt, daß die ausgewählte Verbindung in einem zusätzlichen Test getestet wird, um von der Verbindung induziertes Herauswachsen von Neuriten zu messen.

12. Verfahren nach Anspruch 10, **dadurch gekennzeichnet, daß** das Bestimmen, ob eine Verbindung den Aufbau des Steroidrezeptorkomplexes stört, das Bestimmen umfaßt, ob die Verbindung p23-, FKBP-52- oder hsp-90-Disassoziation vom Steroidrezeptorkomplex bewirkt.

13. Verfahren nach Anspruch 12, **dadurch gekennzeichnet, daß** das Bestimmen, ob eine Verbindung den Aufbau des Steroidrezeptorkomplexes stört, das Bestimmen umfaßt, ob die Verbindung p23-Disassoziation vom Steroidrezeptorkomplex bewirkt.

14. Verfahren nach Anspruch 12, **dadurch gekennzeichnet, daß** das Bestimmen, ob eine Verbindung den Aufbau des Steroidrezeptorkomplexes stört, das Bestimmen umfaßt, ob die Verbindung FKBP-52-Disassoziation vom Steroidrezeptorkomplex bewirkt.

15. Verfahren nach Anspruch 12, **dadurch gekennzeichnet, daß** das Bestimmen, ob eine Verbindung den Aufbau des Steroidrezeptorkomplexes stört, das Bestimmen umfaßt, ob die Verbindung hsp-90-Disassoziation vom Steroidrezeptorkomplex bewirkt.

16. Verfahren nach Anspruch 10, **dadurch gekennzeichnet, daß** das Bestimmen, ob eine Verbindung den Aufbau des Steroidrezeptorkomplexes stört, das Bestimmen umfaßt, ob die Verbindung p23-, FKBP-52- oder hsp-90-Assoziation mit dem Steroidrezeptorkomplex hemmt.

17. Verfahren nach Anspruch 16, **dadurch gekennzeichnet, daß** das Bestimmen, ob eine Verbindung den Aufbau des Steroidrezeptorkomplexes stört, das Bestimmen umfaßt, ob die Verbindung p23-Assoziation mit dem Steroidrezeptorkomplex hemmt.

18. Verfahren nach Anspruch 16, **dadurch gekennzeichnet, daß** das Bestimmen, ob eine Verbindung den Aufbau des Steroidrezeptorkomplexes stört, das Bestimmen umfaßt, ob die Verbindung FKBP-52-Assoziation mit dem Steroidrezeptorkomplex hemmt.

19. Verfahren nach Anspruch 16, **dadurch gekennzeichnet, daß** das Bestimmen, ob eine Verbindung den Aufbau des Steroidrezeptorkomplexes stört, das Bestimmen umfaßt, ob die Verbindung hsp-90-Assoziation mit dem Steroidrezeptorkomplex hemmt.

20. Verfahren nach Anspruch 10, **dadurch gekennzeichnet, daß** das Bestimmen, ob eine Verbindung den Aufbau des Steroidrezeptorkomplexes stört, das Bestimmen umfaßt, ob die Verbindung die Wechselwirkung von p23, FKBP-52 oder hsp-90 mit dem Steroidrezeptorkomplex hemmt.

21. Verfahren nach Anspruch 20, **dadurch gekennzeichnet, daß** das Bestimmen, ob eine Verbindung den Aufbau des Steroidrezeptorkomplexes stört, das Bestimmen umfaßt, ob die Verbindung die Wechselwirkung von p23 mit dem Steroidrezeptorkomplex hemmt.

22. Verfahren nach Anspruch 20, **dadurch gekennzeichnet, daß** das Bestimmen, ob eine Verbindung den Aufbau des Steroidrezeptorkomplexes stört, das Bestimmen umfaßt, ob die Verbindung die Wechselwirkung von FKBP-52 mit dem Steroidrezeptorkomplex hemmt.

23. Verfahren nach Anspruch 20, **dadurch gekennzeichnet, daß** das Bestimmen, ob eine Verbindung den Aufbau des Steroidrezeptorkomplexes stört, das Bestimmen umfaßt, ob die Verbindung die Wechselwirkung von hsp-90 mit dem Steroidrezeptorkomplex hemmt.

24. Verfahren nach Anspruch 10, **dadurch gekennzeichnet, daß** das Bestimmen, ob eine Verbindung den Aufbau des Steroidrezeptorkomplexes stört, das Bestimmen umfaßt, ob die Verbindung die Assoziation mit hsp-90 hemmt oder die Dissoziation von p23 von hsp-90 fördert.

25. Verfahren nach Anspruch 10, **dadurch gekennzeichnet, daß** das Bestimmen, ob eine Verbindung den Aufbau oder die Funktion des Steroidrezeptorkomplexes stört, das Bestimmen umfaßt, ob die Verbindung sich an eine Geldanamycin-Bindungsstelle bindet.

26. Verfahren nach Anspruch 10, **dadurch gekennzeichnet, daß** das Bestimmen, ob eine Verbindung den Aufbau oder die Funktion des Steroidrezeptorkomplexes stört, das Bestimmen umfaßt, ob die Verbindung die Assoziation von FKBP-52 mit hsp-90 hemmt oder die Dissoziation von FKBP-52 von hsp-90 fördert.

## Revendications

1. Utilisation d'une composition qui dissocie l'assemblage d'un complexe stéroïde récepteur pour la fabrication d'un médicament destiné à la stimulation de la croissance des nerfs, dans laquelle la composition comprend un composé qui est
(a) un analogue structurel de FK506 qui se fixe à FKBP-12 avec une valeur K_{d} supérieure à 1 µM;
(b) une benzoquinone ansamycine ;
(c) un analogue structuré de rapamycine qui se fixe à FKBP-12 avec une valeur K_{d} supérieure à 1 µM ;
(d) un peptide comprenant un ou plusieurs domaines répétltifs de tétratricopeptide ;
(e) un anticorps contre un ou plusieurs composants du complexe stéroide récepteur.

2. Utilisation selon la revendication 1, dans laquelle la composition comprend un facteur neurotrophique additionnel autre que l'un des facteurs de la revendication 1.

3. Utilisation selon la revendication 2, dans laquelle le facteur neurotrophique additionnel est un facteur de croissance des nerfs.

4. Utilisation selon l'une quelconque des revendications 1 à 3, dans laquelle le composé est un analogue structurel de FK506 qui se fixe à FKBP-12 avec une valeur K_{d} supérieure à 1 µM.

5. Utilisation selon l'une quelconque des revendications 1 à 3, dans laquelle le composé est un analogue structurel de FK506 qui se fixe à FKBP-12 avec une valeur K_{d} supérieure à 10 µM.

6. Utilisation selon l'une quelconque des revendications 1 à 3, dans laquelle le composé est une banzoquinone ansamycine.

7. Utilisation selon la revendication 6, dans laquelle la benzoquinone ansamycine est la geldanamycine.

8. Utilisation selon l'une quelconque des revendications 1 à 3, dans laquelle le composé est un peptide comprenant un ou plusieurs domaines répétitifs de tétratricopeptide.

9. Utilisation selon l'une quelconque des revendications 1 à 3, dans laquelle le composé est un anticorps contre un ou plusieurs composants du complexe stéroïde récepteur.

10. Procédé pour effectuer le criblage d'un composé qui stimule la croissance des cellules nerveuses, consistant à :
déterminer si un composé testé dissocie l'assemblage ou la fonction d'un complexe stéroïde récepteur en provoquant la dissociation de p23, FKPB-52 ou hsp-90 d'avec le complexe stéroïde récepteur, inhiber l'association de p23, FKBP-52 ou hsp-90 avec le complexe stéroïde récepteur, ou Inhiber l'interaction de p23, FKPB-52 ou hsp-90 avec le complexe stéroïde récepteur ; et
sélectionner un composé qui dissocie l'assemblage ou la fonction du complexe stéroïde récepteur, de façon à sélectionner ainsi un composé qui stimule la croissance des nerfs.

11. Procédé selon la revendication 10, comprenant en outre le test du composé sélectionné dans un dosage additionnel pour mesurer l'excroissance de neurites induite par le composé.

12. Procédé selon la revendication 10, dans lequel le fait de déterminer si un composé dissocie l'assemblage du complexe stéroïde récepteur comprend le fait de déterminer si le composé provoque une dé-association de p23, FKBP-52 ou hsp-90 d'avec le complexe stéroïde récepteur.

13. Procédé selon la revendication 12, dans lequel le fait de déterminer si un composé dissocie l'assemblage du complexe stéroïde récepteur comprend le fait de déterminer si le composé provoque une dé-association de p23 d'avec le complexe stéroïde récepteur.

14. Procédé selon la revendication 12, dans lequel le fait de déterminer si un composé dissocie l'assemblage du complexe stéroïde récepteur comprend le fait de déterminer si le composé provoque une dé-association de FKBP-52 d'avec le complexe stéroïde récepteur.

15. Procédé selon la revendication 12, dans lequel le fait de déterminer si un composé dissocie l'assemblage du complexe stéroïde récepteur comprend le fait de déterminer si le composé provoque une dé-association de hsp-90 d'avec le complexe stéroïde récepteur.

16. Procédé selon la revendication 10, dans lequel le fait de déterminer si un composé dissocie l'assemblage du complexe stéroïde récepteur comprend le fait de déterminer si le composé inhibe l'association de p23, FKBP-52 ou hsp-90 avec le complexe stéroïde récepteur.

17. Procédé selon la revendication 16, dans lequel le fait de déterminer si un composé dissocie l'assemblage du complexe stéroïde récepteur comprend le fait de déterminer si le composé inhibe l'association de p23 avec le complexe stéroïde récepteur.

18. Procédé selon la revendication 16, dans lequel le fait de déterminer si un composé dissocie l'assemblage du complexe stéroïde récepteur comprend le fait de déterminer si le composé inhibe l'association de FKPB-52 avec le complexe stéroïde récepteur.

19. Procédé selon la revendication 16, dans lequel le fait de déterminer si un composé dissocie l'assemblage du complexe stéroïde récepteur comprend le fait de déterminer si le composé inhibe l'association de hsp-90 avec le complexe stéroïde récepteur.

20. Procédé selon la revendication 10, dans lequel le fait de déterminer si un composé dissocie l'assemblage du complexe stéroïde récepteur comprend le fait de déterminer si le composé inhibe l'interaction de p23, FKBP-52 ou hsp-90 avec le complexe stéroïde récepteur.

21. Procédé selon la revendication 20, dans lequel le fait de déterminer si un composé dissocie l'assemblage du complexe stéroïde récepteur comprend le falt de déterminer si le composé inhibe l'interaction de p23 avec le complexe stéroïde récepteur.

22. Procédé selon la revendication 20, dans lequel le fait de déterminer si un composé dissocie l'assemblage du complexe stéroïde récepteur comprend le fait de déterminer si le composé inhibe l'interaction de FKBP-52 avec le complexe stéroïde récepteur.

23. Procédé selon la revendication 20, dans lequel le fait de déterminer si un composé dissocie l'assemblage du complexe stéroïde récepteur comprend le fait de déterminer sl le composé inhibe l'interaction de hsp-90 avec le complexe stéroïde récepteur.

24. Procédé selon la revendication 10, dans lequel le fait de déterminer si un composé dissocie l'assemblage du complexe stéroïde récepteur comprend le fait de déterminer si le composé inhibe l'association ou favorise la dissociation de p23 d'avec hsp90.

25. Procédé selon la revendication 10, dans lequel le fait de déterminer si un composé dissocie l'assemblage ou la fonction du complexe stéroïde récepteur comprend le fait de déterminer si le composé se fixe à un site de fixation de geldanamycine.

26. Procédé selon la revendication 10, dans lequel le falt de déterminer si un composé dissocie l'assemblage ou la fonction du complexe stéroïde récepteur comprend le fait de déterminer si le composé inhibe l'association ou favorise la dissociation de FKBP-52 d'avec hsp-90.
